# EUROPÄISCHE PATENTSCHRIFT

(11) **EP 2 585 005 B1**
(45) Veröffentlichungstag und Bekanntmachung des Hinweises auf die Patenterteilung: **02.12.2015**
(21) Anmeldenummer: 11732372.5
(22) Anmeldetag: 28.06.2011
(51) Int. Cl.: A61F 2/91

(54) **BEFESTIGUNGSMITTEL, IMPLANTAT ZUM IMPLANTIEREN IN DEN MENSCHLICHEN KÖRPER UND VERFAHREN ZUM HERSTELLEN DESSELBEN**
FASTENING MEANS, IMPLANT FOR IMPLANTATION IN THE HUMAN BODY, AND METHOD FOR PRODUCING SAME
MOYEN DE FIXATION, IMPLANT DESTINÉ À ÊTRE IMPLANTÉ DANS UN CORPS HUMAIN ET PROCÉDÉ DE FABRICATION DUDIT MOYEN DE FIXATION

(30) Priorität: 28.06.2010 DE 102010025305
(43) Veröffentlichungstag der Anmeldung: 01.05.2013
(73) Patentinhaber: Admedes Schuessler GmbH, 75179 Pforzheim (DE)
(72) Erfinder: SIEKMEYER, Gerd, 76161 Karlsruhe (DE)
(74) Vertreter: Müller-Boré & Partner Patentanwälte PartG mbB
(86) Internationale Anmeldenummer: PCT/EP2011/003180
(87) Internationale Veröffentlichungsnummer: WO 2012/000653

(56) Entgegenhaltungen:
- EP-A1- 1 000 590
- WO-A2-2007/134290
- US-A- 3 421 997
- US-A- 5 122 242
- US-A- 6 086 599
- US-A1- 2002 120 322
- US-A1- 2006 206 187

## Beschreibung

Die vorliegende Erfindung betrifft einen Stent und Therapieträger, zum Implantieren in den menschlichen Körper und ein Befestigungsmittel sowie ein Verfahren zum Herstellen des Befestigungsmittels.

US 6,086,599 offenbart eine Mikrovorrichtung zum Umpositionieren und Zurückholen von medizinischen Vorrichtungen, die in Blutgefäßen angeordnet sind. Die Mikrovorrichtuna umfasst Formgedächtnispolymerplatten welche mit Haken ausgestaltet sind, und ist an einem Führungsdraht angeordnet. Die Haken der Mikrovorrichtung bilden mit Ösen der medizinischen Vorrichtung eine Verbindungsgeometrie. Mittels Verändert der Temperatur der Haken können die Haken der Mikrovorrichtung mit den Ösen der medizinischen Vorrichtung verbunden oder gelöst werden.

US 2002/0120322 A1 offenbart ein Implantatabgabesystem, wobei ein Implantat an einer Implantatanbindungsstelle mit dem Implantatabaabesystem mittels einer Verriegelungsstruktur, welche komplementäre Verbindungsgeometrien aufweist verbunden ist. Die Verbindungsstrukturen sind als Nut und Holm ausgebildet, so daß das Implantat sich nicht axial unabhängig von dem Implantatabaabesystem bewegten kann. Das Implantat befindet sich in einem röhrenförmigen Element so daß sich der Durchmesser des Implantats nicht ändern kann. Daher bleibt das Implantat mit dem Implantatabgabesystem über die Verbindungsgeometrie verbunden, bis das röhrenförmige Element verschoben wird, wodurch sich der Durchmesser des Implantats wandern kann. Bei Änderung des Durchmessers löst sich das Implantat vom implantatabgabesystem.

WO 2007/134290 A2 offenbart ein Implantatabgabesystem mit einer Verbindungsvorrichtung um sich mit einem Implantate zu verbinden. Mittels Verbindungskonfigurationen wird eine axiale Bewegung des Implantats relativ zum Implantatabgabesystem verhindert, solange der Durchmesser des Implantats sich nicht verändert. Sobald eine Abgabeposition des Implantats erreicht wird, wird der Durchmesser des Implantats vergrößert, so daß sich das Implantat aus der Verbindung mit dem Imalantatabgabesystem löst.

In vielen Anwendungen ist es wünschenswert, ein miniaturisiertes Befestigungsmittel zum Befestigen und insbesondere zum lösbaren bzw. einklipsbaren Befestigen von Bauteilen zu haben. Die Befestigung sollte dabei so gestaltet werden, daß ein Fixieren möglichst ohne Werkzeug erfolgen kann und des weiteren fixierte Bauteile bei Bedarf leicht wieder voneinander gelöst werden können. Derart miniaturisierte Befestigungsmittel sind z.B. in der Uhrenindustrie als sogenannte Snap-in Verbindungen sowie in der Halbleiter und Chip-Industrie wünschenswert, um beispielsweise empfindliche Siliziumchips, Halbleiter oder dergleichen zu transportieren, zu lagern oder handzuhaben.

Biomedizinische Anwendungen betreffen beispielsweise vaskuläre Implantate und Therapieträger zur Langzeitanwendung im menschlichen Körper sowie Clipverbindungen in der Neurochirurgie und Chirurgie. Ein medizinisches Befestigungsmittel muss für eine Implantationsfähigkeit biokompatibel sein und außerdem eine geringe Baugröße und eine ausreichende Festigkeit aufweisen sowie temperaturunempfindlich sein. Zur Anwendung als Therapie- und Medikamententräger werden heute meist metallische Stents als Träger verwendet, die im Anschluss zum Beispiel eine Oberflächenbeschichtung oder Covering/Umhüllung aus Metallen oder Polymerverbindungen erhalten, in die auch ein Medikament eingebettet sein kann. Diese flexiblen Umhüllungen können geflochten, gespritzt oder aus einem dünnen, Lochblech geformt werden. Wünschenswert ist hier eine Verbindungstechnik, die das finale Aussenprofil durch Überstehen von Befestigungsmitteln (z.B. bei Nietköpfen) nicht wesentlich vergrössert.

Zur Therapie bei der Medikamentengabe werden die Stentoberflächen mit Polymeren beschichtet, beispielsweise durch sogenanntes Dip-coating, Spraying von Lacken etc., die über einen Sol-Gel Prozess und/oder reine Adhesions-Kräfte an der metallischen Stentoberfläche halten. Darüber hinaus werden Stentoberflächen nanoporös durch chemische Bearbeitung vergrössert bzw. aufgeraut. Ferner werden auch Polymerscheiben, die mit Medikamenten gefüllt sind, in Ringelemente eingepresst. Andere Therapieformen (z.B. zur Thrombenfilterung) verlangen die Umhüllung mit einem dünnen, meist metallischen Geflecht, maschigem Blech oder Film.

Ein weiterer Lösungsansatz um Stents als Therapieträger zu optimieren sind metallische, offenporöse Gewebe und Materialien zum Einbetten von Medikamenten und Zellen. Diese porösen Materialien werden zum Beispiel durch sintern von Pulvern hergestellt, sind dann aber nicht elektropolierbar und aufgrund der Restoxide nur bedingt biokompatibel oder nicht vaskulär einsetzbar.

Bei einer reinen Oberflächenbeschichtung besteht der Nachteil, dass die Ladungskapazität mit Medikamenten zu gering ist und eine gezielte, lokale Applikation nur sehr bedingt machbar ist.

Es gibt insbesondere kein Reservoir, aus dem die Medikamenten-Abgabe über einen langen Zeitraum gespeist werden kann. Aufgrund der fehlenden Biokompatibilität sind 3D elektropolierte, poröse Strukturen auch nicht realisierbar. Bei der Vewendung weiterer Funktionsbaugruppen am Stent (z.B. Umhüllungen), besteht weiter das Problem der Fixierung dieser Baugruppen.

Bei neuroradiologischen und coronaren Produkten wie z.B. Flow-Divertern oder coronaren Stents aus Nitinol besteht zudem die Notwendigkeit der reversiblen Applikation der Therapieträger bzw. der Möglichkeit einer kontinuierlichen Neupositionierung. Hierbei müssen lösbare Verbindungselemente zwischen Stent und Applikations- bzw. Transportdrähten vorhanden sein.

Die Aufgabe der Erfindung besteht somit in der Schaffung eines miniaturisierten Befestigungsmittels, das ohne Werkzeug bei Bedarf gelöst und wieder befestigt werden kann. Darüber hinaus sollte das Befestigungsmittel eine hohe Festigkeit aufweisen und biokompatibel sein.

Diese Aufgabe wird durch die Merkmale der unabhängigen Ansprüche gelöst. Vorteilhafte Weiterbildungen der Erfindung sind in den abhängigen Ansprüchen definiert.

Gemäß einem Gesichtspunkt wird ein Stent und Therapieträger, zum Implantieren oder Anwenden in den/am menschlichen Körper mit einem Substrat aus Nitinol für biokompatible und/oder hochfeste Mikro-Verbindungen zur Verfügung gestellt, wobei eine Vielzahl von hinterschnittenen Vorsprüngen oder Stiften etc. von dem Substrat hervorragt, um ein Verhaken der hinterschnittenen Vorsprünge oder Stifte mit einem entsprechenden Gegenstück zu ermöglichen, so dass das Befestigungsmittel lösbar mit dem Gegenstück verbindbar ist.

Aufgrund der hinterschnittenen Vorsprünge und den superelastischen Werkstoffeigenschaften des Nitinol kann ein Verhaken mit einem Gegenstück auf einfache Weise ähnlich wie bei einer Velcro^{®}-Verbindung erfolgen, ohne dass es zu einer plastischen Verformung der Stifte beim Verhaken kommt. Das Gegenstück kann dabei ähnlich wie bei der Velcro^{®}-Verbindung ein Geflecht aus unterschiedlichen Fasern sein. Es ist jedoch auch möglich als Gegenstück ein ähnliches oder gleiches Befestigungsmittel mit hinterschnittenen Vorsprüngen zu verwenden, weil aufgrund der Elastizität des Nitinols die hinterschnittenen Vorsprünge eines Substrats mit den hinterschnittenen Vorsprüngen des anderen Substrats in Eingriff bringbar sind. Derart kann ein lösbares Befestigungsmittel bestehend aus zwei gleichartigen Substraten geschaffen werden, um eine Vielzahl unterschiedlicher Bauteile zu verringern. Somit werden Fertigungskosten und ein Aufwand für die Herstellung des Befestigungsmittels verringert.

Vorzugsweise weist das Befestigungsmittel bzw. der Stent und Therapieträger zumindest ein Paar Substrate mit einer Vielzahl von hinterschnittenen Vorsprüngen auf, die so gestaltet sind, dass die beiden Substrate lösbar miteinander verbindbar sind, indem die Vorsprünge eines Substrats mit den Vorsprüngen des anderen Substrats miteinander verhaken.

Weiter bevorzugt weist zumindest eine Gruppe der Vorsprünge wenigstens teilweise einen im wesentlichen kreisförmigen Querschnitt auf und/oder es ist zumindest eine Gruppe der Vorsprünge im wesentlichen pilzförmig geformt.

Weiter bevorzugt weist zumindest eine Gruppe der Vorsprünge zumindest einen Abschnitt kleinen Querschnitts und zumindest einen Abschnitt großen Querschnitts auf, wobei der Abschnitt kleinen Querschnitts zwischen dem Substrat und dem Abschnitt großen Querschnitts angeordnet ist.

Weiter bevorzugt ist ein Rand des Abschnitts großen Querschnitts abgerundet und/oder angeschrägt/angefast und/oder konisch. Aufgrund der Abrundung bzw. Anschrägung bzw. Fase bzw. konischen Form des Abschnitts großen Querschnitts wird ein Ineingriffbringen der Vorsprünge eines Substrats mit den Vorsprüngen des anderen Substrats erleichtert.

Vorzugsweise sind die Vorsprünge in einem Abstand von etwa 50 bis etwa 2000 µm, vorzugsweise in einem Abstand von etwa 100 bis etwa 300 µm voneinander an dem Substrat angeordnet.

Weiter bevorzugt hat der Abschnitt großen Durchmessers einen Durchmesser von etwa 20 bis etwa 300 µm, vorzugsweise von etwa 50 bis etwa 200 µm und/oder der Abschnitt kleinen Durchmessers hat einen Durchmesser von etwa 1 bis etwa 300 µm, vorzugsweise von etwa 40 bis etwa 150 µm.

Gemäß einem weiteren Gesichtspunkt wird ein Befestigungsmittel mit einem Substrat aus Nitinol für biokompatible und/oder hochfeste Mikro-Verbindungen bereitgestellt, wobei zumindest ein hinterschnittener Vorsprung von dem Substrat hervorragt, um ein Verhaken des hinterschnittenen Vorsprungs mit einem entsprechenden Gegenstück zu ermöglichen, so dass das Befestigungsmittel lösbar mit dem Gegenstück verbindbar ist.

Vorzugsweise ist das Befestigungsmittel zumindest ein Abschnitt oder ein Teilbereich eines Stents oder eines medizinischen Produkts, welches in einen menschlichen Körper eingeführt bzw. positioniert werden kann.

Vorzugsweise umfasst das Befestigungsmittel zusätzlich zumindest einen Kopplungsbereich, so dass ein Vorsprung-Gegenstück, welches ebenfalls zumindest einen Vorsprung umfasst, mittels des Vorsprungs mit dem Kopplungsbereich des Befestigungsmittels verbindbar ist, wobei der Vorsprung des Vorsprung-Gegenstücks in den Kopplungsbereich des Befestigungsmittels eingreift.

Weiterhin vorzugsweise ist der Kopplungsbereich des Befestigungsmittels als zylindrische und/oder konische Durchführung und/oder als Sackloch ausgebildet, wobei der Vorsprung des Vorsprung-Gegenstücks in den Kopplungsbereich des Befestigungsmittels eingreift, wenn das Befestigungsmittel mit dem Vorsprung-Gegenstück verbunden ist.

Weiterhin vorzugsweise ist das Befestigungsmittel als Brückenabschnitt ausgebildet, so dass der hinterschnittene Vorsprung in den Kopplungsbereich eines Hülsen-Gegenstücks eingreift, wenn der Brückenabschnitt mit dem Hülsen-Gegenstück verbunden ist, wobei das Fortsatz-Gegenstück als Hülse bzw. Hülsenabschnitt ausgebildet ist.

Weiterhin vorzugsweise umfasst das Befestigungsmittel einen Fortsatz, wobei der Fortsatz eine Vielzahl von hinterschnittenen Vorsprüngen aufweist, welche umfänglich an der Mantelfläche des Fortsatzes angeordnet sind, so dass die hinterschnittenen Vorsprünge in den Kopplungsbereich eines Fortsatz-Gegenstücks eingreifen, wenn der Fortsatz mit dem Fortsatz-Gegenstück verbunden ist, wobei das Fortsatz-Gegenstück als Hülse ausgebildet ist.

Weiterhin vorzugsweise ist das Befestigungsmittel als Verbindungsplatte ausgebildet und umfasst eine Vielzahl von Vorsprüngen, und wobei ein Verbindungsplatten-Gegenstück ebenfalls eine Vielzahl von Vorsprüngen aufweist, wobei die Vorsprünge des Befestigungsmittels und des Verbindungsplatten-Gegenstücks derart zueinander angeordnet sind, dass das Befestigungsmittel mit dem Verbindungsplatten-Gegenstück durch Verhaken der Vorsprünge ineinander verbindbar ist.

Gemäß einem weiteren Gesichtspunkt hat ein Implantat zum Implantieren in den menschlichen Körper zumindest ein derartiges Befestigungsmittel, wie vorstehend beschrieben.

Gemäß einem weiteren Gesichtspunkt wird ein Verfahren zum Herstellen eines Befestigungsmittels mit den folgenden Schritten geschaffen:
Bereitstellen eines Substrats aus Nitinol; und elektrochemische Bearbeiten einer Oberfläche des Substrats zum Erzeugen einer Vielzahl von hinterschnittenen Vorsprüngen an der Oberfläche des Substrats.

Vorzugsweise erfolgt die elektrochemische Bearbeitung mit Hilfe eines Elektrodenfelds, wobei Elektroden des Elektrodenfelds isolierte Seitenflanken aufweisen oder aus dünnen Blechen (<100 Mikrometer) auf Polymerträgern (zum Beispiel kupferkaschierte Laminate) geformt werden.

Die Erfindung wird nun anhand bevorzugter Ausführungsbeispiele unter Bezugnahme auf die beigefügten Zeichnungen näher erläutert.
Fig. 1 zeigt ein erfindungsgemäßes Substrat mit einer Vielzahl von Vorsprüngen.
Fig. 2 zeigt das Substrat von Fig. 1, wenn die Vorsprünge des Substrats mit Vorsprüngen eines gleichartigen anderen Substrats in Eingriff gebracht sind.
Fig. 3 zeigt eine Schnittansicht eines Elektrodenfelds zum Herstellen der Vorsprünge an dem Substrat.
Fig. 4 zeigt einen Stentsteg mit Vorsprüngen zum Einklipsen eines Medikaments.
Fig. 5 zeigt eine Stentstütze mit einem Trägerblech oder Filter.
Fig. 6 zeigt einen Stentsteg mit Vorsprüngen, die in Vertiefungen eines porösen Medikamentenbehälters eingeklipst werden.
Fig. 7 zeigt eine Oberflächenabwandlung von einer makroskopischen Erhöhung der Oberfläche zu einer Nanooberfläche.
Figuren 8a bis 8c zeigen einen Ausschnitt einer Stentstruktur, in welche ein Therapieträger einsetzbar ist.
Fig. 9 zeigt Brückenabschnitte einer Stentstruktur, zwischen welchen eine Hülse anordenbar ist.
Fig. 10 zeigt eine schematischen Ausschnitt einer Stentstruktur, welche zwei Verbindungselemente und eine Hülse umfaßt.
Fig. 11 zeigt einen Ausschnitt einer Stentstruktur in Form eines Multifilament-Geflechts.
Figs. 12a bis 12f zeigen eine weitere Ausgestaltung von Brückenabschnitte einer Stentstruktur und einer Hülse zum Verbinden der Brückenabschnitte.
Fig. 13 zeigt eine vereinfachte Darstellung eines Zuführsystems mit einem Verbindungselement.
Fig. 14 zeigt ein medizinisches Produkt mit einer Verbindungsplatte.
Fig. 15 zeigt einen Ausschnitt eines mehrgliedrigen Verbindungselements mit Verbindungsplatten.
Fig. 16 zeigt einen vergrößerten Ausschnitt des Zuführsystems aus Fig. 13.
Fig. 17 zeigt ein Zuführsystem mit einem daran angekoppelten Stent.
Fig. 18 zeigt einen vergrößerten Ausschnitt von verklippsten Verbindungsplatten.
Fig. 19 zeigt eine Variante einer Verbindungsplatte.
- Fig. 20 zeigt eine weitere Variante einer Verbindungsplatte.
Fig. 21 zeigt verklippste Verbindungsplatten.
Fig. 22 zeigt einen vergrößerten Ausschnitt der Fig. 21.
Fig. 23 zeigt eine weitere Ausführung einer Verbindungsplatte.
Fig. 24 zeigt eine weitere Ausführung einer Verbindungsplatte.
Fig. 25 zeigt mehrere ineinander verklippste Verbindungsplatten in einer Seitenansicht.
Fig. 26 zeigt eine perspektivische Ansicht der Fig. 25.
Fig. 27 zeigt eine perspektivische Ansicht von verklippsten Verbindungsplatten.
Fig. 28a bis 30b zeigen verschiedene Multi-Lumen-Hülsen

Stents werden heute zur Behandlung von vaskulären Stenosen oder als Medikamenten- und Therapieträger z.B. für die Behandlung von Krebs verwendet. Vorzugsweise sollen hier nur biokompatible, metallische Werkstoffe in Verbindung mit einem Medikament oder einer funktionalen Therapie, wie Filtergeflechten verwendet werden, die sich minimal invasiv anwenden und applizieren lassen. Nitinol ist für eine solche Anwendung aufgrund seiner Werkstoffeigenschaften prädestiniert.

Bei Medikamentenanwendungen besteht z.B. das Problem der lokalen, gezielten Medikamentenapplikation einer bestimmten Medikamenten-Menge, über einen langen Zeitraum. Zusätzlich soll die Freisetzungsrate langsam über einen längeren Zeitraum oder variabel einstellbar sein. Vorzugsweise sollen hier nur biokompatible, metallische Werkstoffe in Verbindung mit einem Medikament zum Einsatz kommen. Bei der Verwendung als funktionaler Therapieträger (Stent & Umhüllung) besteht zusätzlich das Problem der Verbindungstechnik zwischen zwei individuellen Baugruppen mit unterschiedlichen Aufgaben.

Nachfolgend wird die Möglichkeit einer Clip-Technologie beschrieben und deren mögliche Anwendungen auf Implantate aus Nitinol die eine Lösung dieser genannten Fünktionsaufgaben von Stents erlaubt.

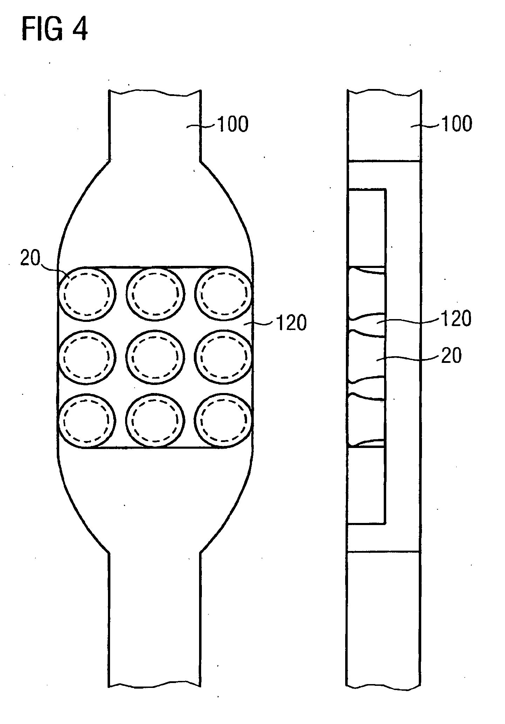

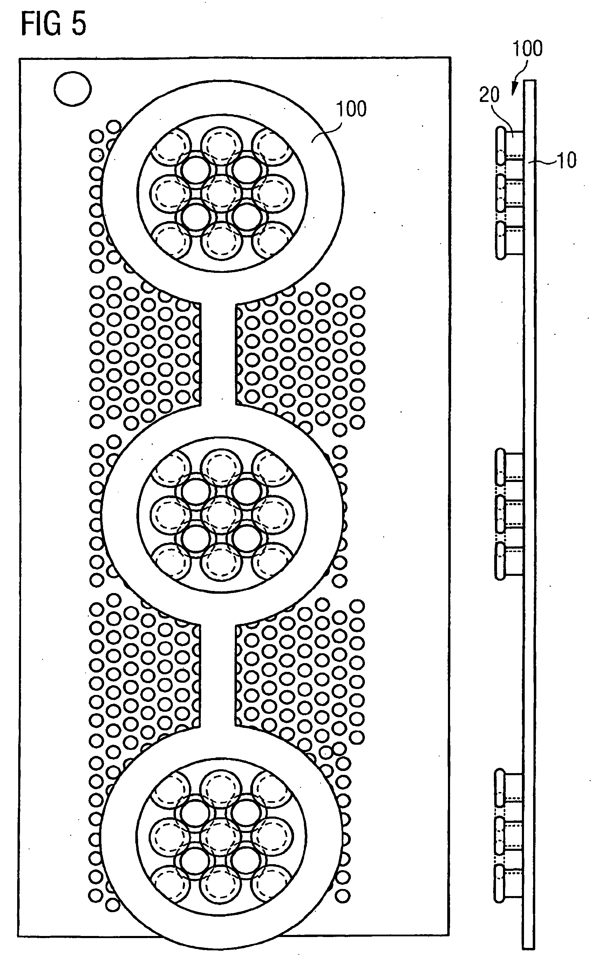

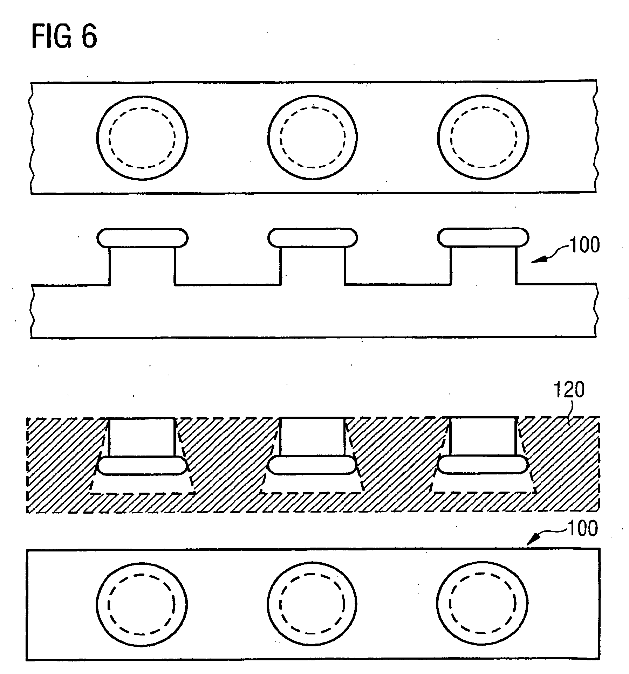

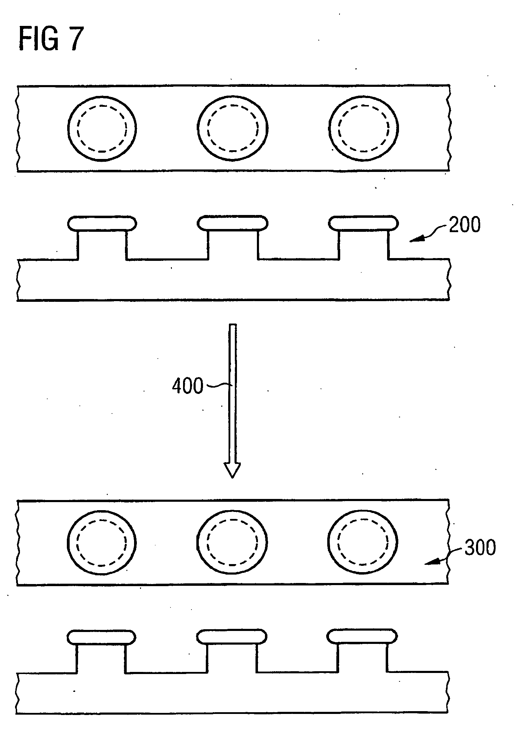

Die Micro-Clip Technologie wird beispielsweise verwendet:
- Zum Anclipsen oder Einbetten einer grösseren Medikamentenschicht über ein Polymer, wie in Fig. 4 gezeigt ist;
- Zum Fixieren einer grossen, porösen Medikamenten-Trägermatrix (drug Container) auf dem Stent ohne mechanische Beeinflussung des Implantates, wie in Fig. 6 gezeigt ist;
- Zum "Deckeln" eines Medikamentbehälters der nur bedingt biokompatibel ist.
- Zur gezielten, lokalen Applikation und Applizieren eines Medikamentes oder einer Therapieform über einen langen Zeitraum, wie in Fig. 5 gezeigt ist;
- Zur Kombination von makroskopischen und mikroskopischen Oberflächeneigenschaften im Mikrometer- und Nanometer-Massstab, wie in Fig. 7 gezeigt ist;
- Zur Herstellung biomimetrischer Oberflächen auch mit Kombination aus Zellstimulation und Medikamentengabe;
- Zur Fixierung eines porösen oder dünnen Nitinol Netzes/Geflechtes/Films auf einem Stentträger, wie in Fig. 5 gezeigt ist.
- Zum lösbaren Verbinden bei der Applikation des Therapieträgers bei z.B. neurologischen oder coronaren Flow-Divertern und Stents

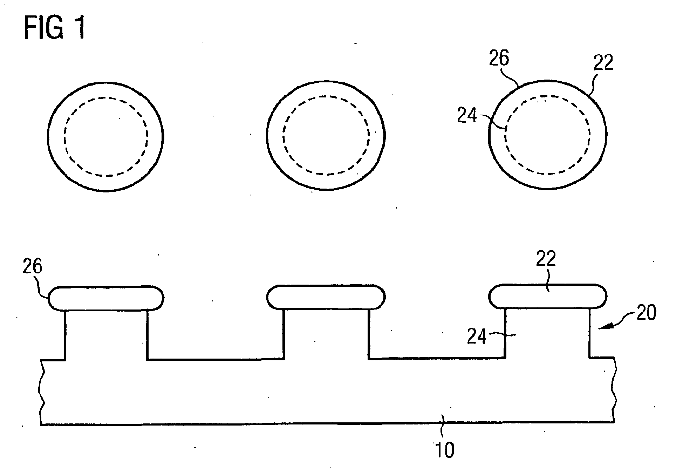

Wie in Fig. 1 gezeigt ist, ragen von dem Substrat 10 aus Nitinol eine Vielzahl von Vorsprüngen 20 oder Stengel oder dergleichen hervor, die eine im wesentlichen pilzförmige oder hakenförmige Gestalt aufweisen. Die pilzförmige Gestalt der Vorsprünge 20 umfaßt beispielsweise einen Abschnitt großen Durchmessers 22 an einem distalen Ende des Vorsprungs 20 sowie einen Abschnitt kleinen Durchmessers 24 an einem proximalen Ende des Vorsprungs 20 bzw. zwischen dem Abschnitt großen Durchmessers 22 und dem Substrat 10. Derart bildet der Abschnitt kleinen Durchmessers 24 eine hinterschnittene Form des Vorsprungs 20, um mit einem entsprechenden Gegenstück in Eingriff zu treten.

Die Erfindung ist jedoch nicht auf die Pilzform der Vorsprünge 20 beschränkt, sondern es können auch andere Formen eingesetzt werden so lange wie diese wenigstens teilweise eine hinterschnittene Form haben, um mit einem entsprechenden Gegenstück formschlüßig in Eingriff zu treten. Beispielsweise können die Vorsprünge 20 eine Hakenform, eine flachen oder einen gewölbten Kopf, einen elliptischen oder dreieckigen Querschnitt oder dergleichen haben. Eine eckige Kopfgeometrie wie beispielsweise hexagonal ist ebenfalls möglich. Auch eine im wesentliche runde Tellerform ist möglich.

Ein Stent 100 zum Implantieren in den menschlichen Körper weist ein derartiges Substrat mit Vorsprüngen 20 auf, wie in den Figuren 4 bis 7 gezeigt ist. Dabei können die Vorsprünge 20 zum Einklipsen in eine gegenüberliegende Stentstruktur oder zum Halten und Befestigen eines Medikaments 120 verwendet werden. Dieses Medikament 120 wird somit zusammen mit dem Stent 100 in den menschlichen Körper implantiert, um dort in einem vorgegebenen Zeitraum an den menschlichen Körper abgegeben zu werden.

Das Medikament 120 kann dabei die Gestalt einer mit Öffnungen versehenen Tablette haben, um in die Vorsprünge 20 des Stents 100 eingeklipst zu werden.

Das Verhältnis aus Kopfdurchmesser (Abschnitt großen Durchmessers 22) zu Stengeldurchmesser (Abschnitt kleinen Durchmessers 24) beträgt bevorzugt etwa 1:0.3 bis zu etwa 1:0,9, am besten etwa 1:0,6 bis etwa 1:0,8.

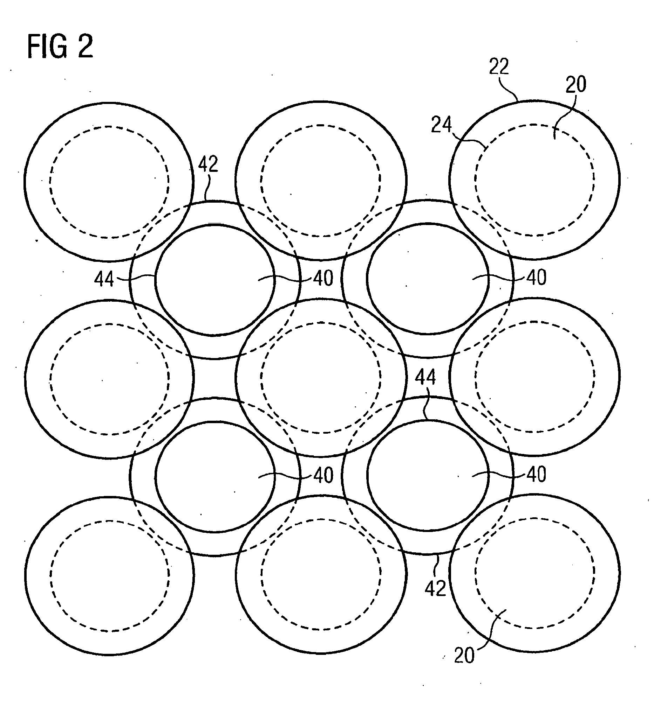

Das Gegenstück kann dabei ähnlich wie bei einer Velcro^{®}-verbindung ein Geflecht aus unterschiedlichen Fasern sein. Es kann jedoch vorzugsweise auch ein gleichartiges Substrat 10 mit Vorsprüngen 20 bzw. 40 sein, so daß die Vorsprünge 20 mit Vorsprüngen 40 in Eingriff treten, wie dies in Fig. 2 dargestellt ist.

Das Ineingrifftreten der Vorsprünge 20 des einen Substrats mit Vorsprüngen 40 des anderen Substrats erfolgt durch Aufeinanderdrücken des einen Substrats auf das andere Substrat, um dadurch ein elastisch rückstellfähiges Verbiegen der Vorsprünge 20, 40 derart zu veranlassen, daß die Abschnitte großen Durchmessers 22, 42 jeweils in die durch die Abschnitte 24, 44 kleinen Durchmessers gebildeten Zwischenräume eintreten. Derart werden zwei gleichartige Substrate miteinander verhakt und können durch Auseinanderziehen wieder voneinander gelöst werden.

Um ein Ineingrifftreten der Vorsprünge 20 mit den Vorsprüngen 40 zu erleichtern, hat zumindest eine Gruppe von Vorsprüngen einen Rand 26, der konisch, abgerundet, abgefast oder dergleichen ist.

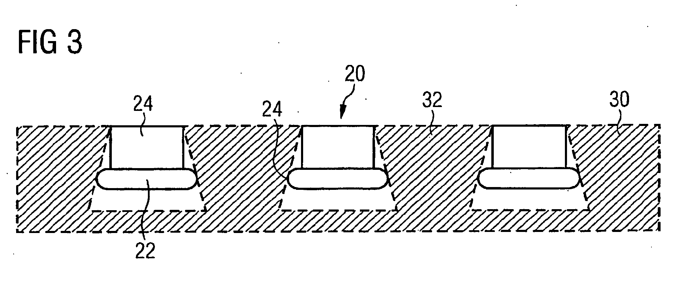

In Fig. 3 ist ein Elektrodenfeld 30 mit Elektroden 32 zum Herstellen der Vorsprünge 20 dargestellt. Dieses Elektrodenfeld 30 wird beispielsweise aus einer Graphitplatte erzeugt und hat im wesentlichen konische Vertiefungen zum Erzeugen der hinterschnittenen Vorsprünge 20 mit dem Abschnitt großen Durchmessers 24 und dem Abschnitt kleinen Durchmessers 24. Durch die Isolierung von Seitenflanken der Elektroden 32 lassen sich verschiedene Abtragsparameter während der Tiefenbearbeitung ansteuern. Somit kann ein vorteilhaftes Hinterschneiden der Vorsprünge 20 verwirklicht werden.

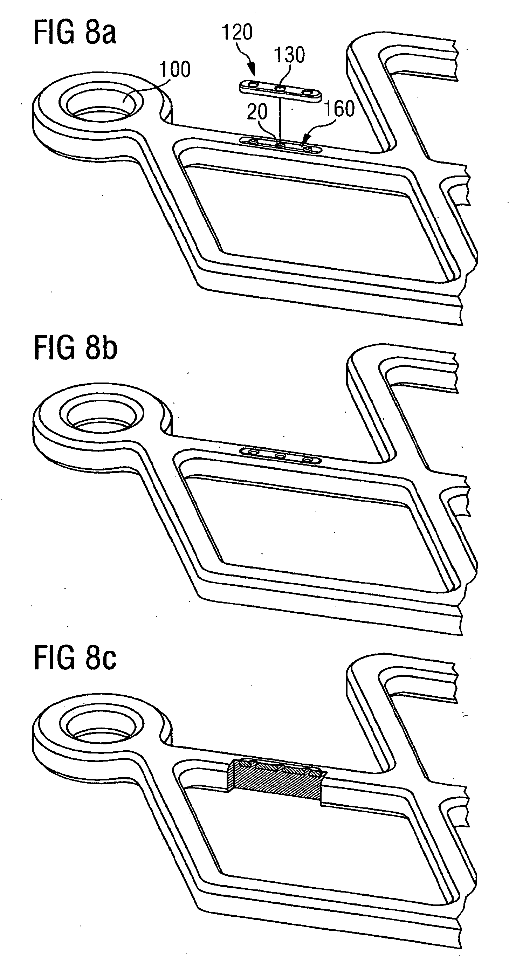

**Figs. 8a bis 8c** zeigen einen Ausschnitt einer Stentstruktur bzw. eines Stents 100, in welchen ein Medikamenten- oder Therapieträger bzw. Medikament 120 einklippsbar ist. Um den Medikamententräger 120 mit dem Stent 100 verklippsen zu können, umfaßt der Stent einerseits Vorsprünge in Form von makroskopischen Erhöhungen 200 und der Medikamententräger 120 als Gegenstück zu den Vorsprüngen ausgebildete Kopplungsbereiche 130. Wie in Fig. 8a gezeigt, sind die Vorsprünge 20 in einer nut-förmigen Ausnehmung 160, welche an einem Steg der Stentstruktur 100 ausgebildet ist, angeordnet. Entsprechend ragen die Vorsprünge vorzugsweise nicht von der Stentstruktur bzw. des Stent-Stegs hervor. Alternativ kann die Ausnehmung 160 auch eine andere Form als eine nut-förmige aufweisen.

Vorzugsweise ist der Medikamententräger 120 als positives Gegenstück zur Ausnehmung 160 mit den Vorsprüngen 20 des Stents 100 ausgebildet, so daß sich nach einem Einklippsen bzw. Einsetzen des Medikamententrägers 120 in die Stentstruktur 100 eine im wesentlichen kontinuierliche Oberflächengestaltung des Stents ergibt. In anderen Worten wird die Ausnehmung 160 des Stents 100 durch Einsetzen des Medikamententrägers 120 im wesentlichen ausgefüllt bzw. abgedeckt. Insbesondere dient die Stentstruktur 100 bzw. der Abschnitt der Stentstruktur, der die Ausnehmung 160 zusammen mit Vorsprüngen 20 umfasst als Befestigungsmittel.

Während Fig. 8a einen Abschnitt eines Stents 100 vor dem Einsetzen eines Medikamententrägers 120 zeigt, zeigt Fig. 8b einen bereits eingesetzten bzw. verklippsten Medikamententräger 120.

Fig. 8c zeigt eine Teilschnittansicht der Stentstruktur 100 mit einem verklippsten Medikamententräger 120, wie in Fig. 8b dargestellt. Wie der Fig. 8c zu entnehmen ist, wird der Medikamententräger 120 durch die Vorsprünge 20 des Stents 100 sicher an dem Stent befestigt bzw. von den Vorsprüngen 20 gehalten.

Vorzugsweise sind die Vorsprünge wie vorhergehend beschrieben ausgebildet. Insbesondere können diese Vorsprünge auch wie die vorangegangenen beschriebenen Vorsprünge 40 ausgebildet sein.

Die Kopplungsbereiche 130 des Medikamententrägers 120, in welchen die Vorsprünge 20 des Stents 100 positioniert werden können, können als zylindrische und/oder konische Durchführungen ausgebildet sein, welche sich von einer Seitenfläche zu einer gegenüberliegenden Seitenfläche des Medikamententrägers 120 erstreckt. Alternativ können in dem Medikamententräger 120 Sacklöcher ausgebildet sein, welche geeignet sind, die Vorsprünge aufzunehmen, um eine sichere Verbindung mit dem Stent bereitzustellen. Insbesondere können die Kopplungsbereiche 130 Aufweitungen oder Hinterschnitte aufweisen, welche geeignet sind, Vorsprünge der vorangegangenen Art aufzunehmen.

Entsprechend kann die Stentstruktur 100 oder ein Teil der Stentstruktur 100 ein Substrat aus Nitinol sein, welches ein oder mehrere Vorsprünge 20 umfasst, so dass die Vorsprünge 20 sich mit dem Medikamententräger 120, welches als Gegenstück fungiert, verhaken kann um eine hochfeste Mikro-Verbindung einzugehen bzw. bereitzustellen.

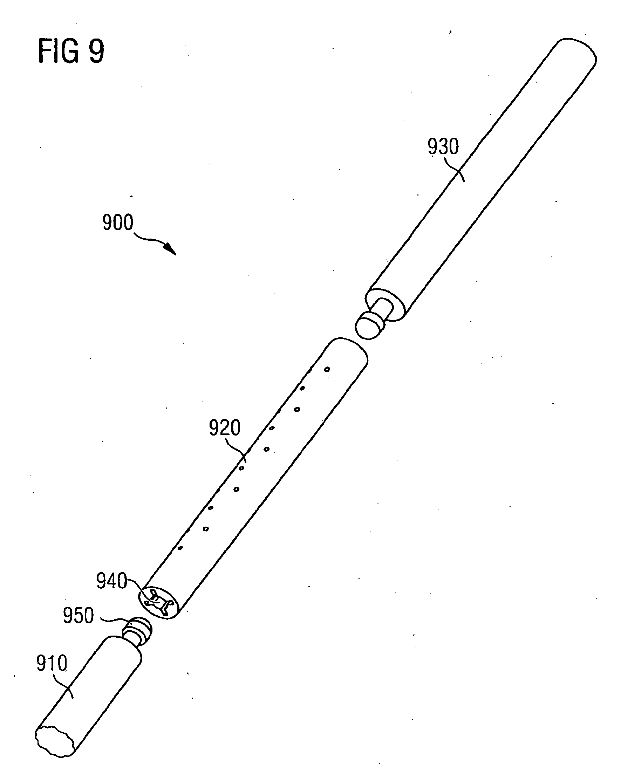

**Fig. 9** zeigt ein Stecksystem 900, welches zwei Brückenabschnitte/-teile 910 und 930 einer Stentstruktur umfaßt, zwischen welchen eine Hülse 920 anordenbar ist.

Die Hülse 920 kann eine Medikamentenhülse sein, in welche Medikamente zur Abgabe in Körpergewebe eingebracht sind. Auch kann die Hülse 920 aus röntgensichtbarem Material ausgebildet sein. Die Hülse 920 umfaßt zwei Kopplungsbereiche 940, in welche jeweils ein Vorsprung 950 eines Stent-Brückenabschnittes 910 einsetzbar bzw. einklippbar ist. Die Vorsprünge 950 können vorzugsweise wie die vorangegangen beschriebenen Vorsprünge 20 bzw. 40 ausgebildet sein. Ebenso können die Kopplungsbereiche 940 wie vorangehend beschriebene Kopplungsbereiche zur Aufnahme eines Vorsprungs ausgebildet sein.

Die in Fig. 9 gezeigten Stent-Brückenabschnitte bzw. Brückenabschnitte 910 sind im wesentlichen als Zylinder ausgebildet, welche zumindest an einer Seitenfläche einen Vorsprung 950 aufweisen. Die Hülse 920 weist ebenfalls eine zylindrische Form auf, so daß in einem bevorzugten Ausführungsbeispiel die Abmaße, insbesondere der Durchmesser der zylindrischen Stent-Brückenabschnitte 910 und der Hülse 920 identisch oder sehr ähnlich sind. Zumindest kann vorgesehen sein, daß in einem Übergangsbereich von Stent-Brückenabschnitt zu Hülse ein weitgehend versatzfreier Übergang geschaffen wird. Dies hat den Vorteil, daß kein oder nur ein geringer Versatz in den Übergängen von einem Stent-Brückenabschnitt 910 zu der Hülse 920 vorliegt. Hierdurch kann beispielsweise eine Verletzung von Körpergewebe sowie ein Verhaken oder Verklemmen bei einem Einführen oder Positionieren eines Stents, welcher solche Brückenabschnitte und medizinische Hülsen umfaßt, verhindert werden.

In weiteren Ausführungsbeispielen kann vorgesehen sein, daß die Brückenabschnitte als auch die medizinische Hülse andere geometrische Formgebungen aufweisen. Beispielsweise könnte die medizinische Hülse nicht als "gerader" Zylinder ausgebildet sein, sondern eine Biegung und/oder einen Knick aufweisen, so daß die Seitenflächen der medizinischen Hülse an welchen die Kopplungsbereiche 940 angeordnet sind, nicht planparallel gegenüberliegen bzw. gegenüberstehen, sondern schief zueinander angeordnet sind. Auch könnte die medizinische Hülse in einer Ausführungsform, derart gebogen sein, daß ihre Längsachse einem bestimmten Radius entspricht oder eine Kurve beschreibt.

In einem weiteren Beispiel, könnten die Stent-Brückenabschnitte 910 derart geformt sein, daß sie Teile eines Stent-Stegs, wie in Figs. 8a bis 8c entsprechen, wobei anstatt einer, wie in der Fig. 8a gezeigten, Ausnehmung zur Aufnahme eines Medikaments ein ganzes Stück des Stent-Stegs herausgenommen bzw. ausgespart wird, um analog, wie in Fig. 9 gezeigt, eine Hülse 920 einsetzen zu können.

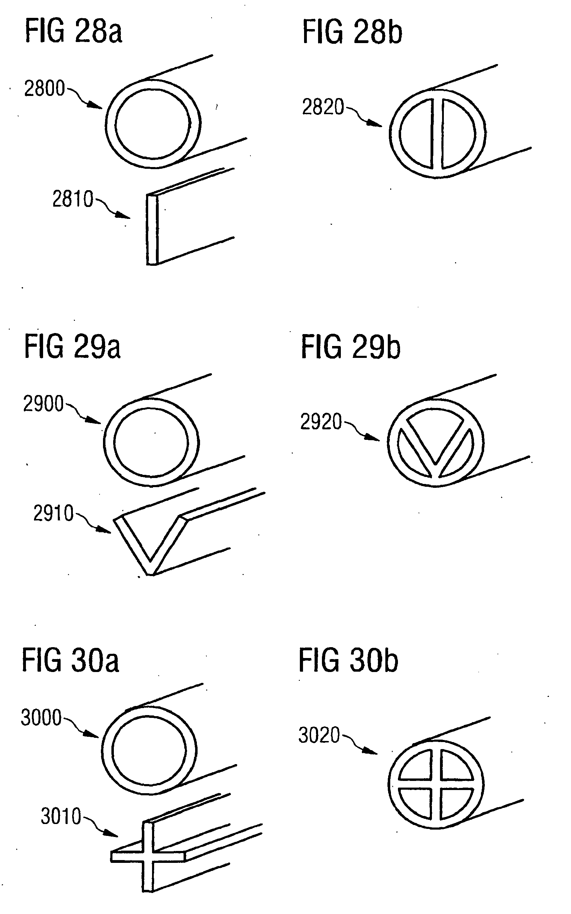

In einem weiteren Beispiel kann zumindest einer der Kopplungsbereiche 940 als Medikamententräger ausgebildet sein, wobei der Kopplungsbereich dann als Multi-Lumen-Hülse 2820, 2920, 3020, wie in Figuren 28a bis 30b gezeigt, geformt sein kann. Wie in den Figuren 28a bis 30b gezeigt, kann ein Hohlzylinder bzw. ein Rohrmaterial 2800, 2900, 3000 verwendet werden, in welches ein oder mehrere Bleche bzw. Plattenmaterial 2810, 2910, 3010 Trennwände eingezogen werden können, um Kammern gleicher oder verschiedener Größe zu bilden. Anstatt Bleche 2810, 2910, 3010 als Trennwände einzusetzen, kann alternativ Material aus einem Zylinder bzw. Vollmaterial herausgenommen werden, um Kammern zu formen. Beispielsweise könnten solche Kammern durch Laserbearbeitung geformt werden. In die entstandenen Kammern können dann Medikamente eingebracht werden. Vorzugsweise können in Multi-Lumen-Hülsen verschiedene Medikamente eingebracht werden, so dass verschiedene Kammern für unterschiedliche Medikamente vorgesehen werden können. Auch kann bei Multi-Lumen-Hülsen mit Kammern verschiedener Größe (siehe beispielsweise die Multi-Lumen-Hülse 2920) die Menge der Medikamentengabe durch die Auswahl der Kammer bestimmt bzw. festgelegt werden.

Die Kopplungsbereiche 940 der Medikamentenhülse 920 können vorzugsweise mit einem UKP-Laser geformt/gebohrt werden. In einer weiteren Ausbildung können diese Kopplungsbereiche 940 ebenfalls, wie vorstehend beschrieben, Hinterschneidungen aufweisen.

Die Hülse 920 dient insbesondere als Drug-Container, um Medikamente oder Therapiemittel zu tragen. Beispielsweise kann die Hülse 920 auch teilweise oder sogar vollständig aus einem Material hergestellt sein, daß beispielsweise zur thermischen Therapie von Körpergewebe wie beispielsweise bei der Krebsbehandlung, verwendet werden kann.

Entsprechend können die Stent-Brückenabschnitte oder ein Teil der Stent-Brückenabschnitte jeweils oder gemeinsam ein Substrat aus Nitinol sein, welches ein oder mehrere Vorsprünge umfasst. Zusätzlich oder alternativ kann die Hülse oder ein Teil der Hülse ein Substrat aus Nitinol sein, welches ein oder mehrere Vorsprünge umfasst. Weiterhin können die beschriebenen Stent-Brückenabschnitte auch Brückenabschnitte von anderen mikrostrukturierten Bauteilen bzw. medizinischen Produkten sein, welche zur medizinischen Anwendung im menschlichen Körper gedacht sind. Beispielsweise könnte ein Brückenabschnitt ein Abschnitt eines Zuführsystems sein, welches zum Einführen und Positionieren von medizinischen Produkten verwendet wird. Insbesondere können Werkstoffe wie 316LVM und/oder CoCr-Legierungen verwendet werden. Weiterhin zusätzlich oder alternativ können Hülsen (wie beispielsweise die Hülse 920) oder auch Multi-Lumen-Hülsen (wie beispielsweise die Hülsen 2820 bis 3020), Stent-Brückenabschnitte oder Teile der Stent-Brückenabschnitte zumindest teilweise aus einem harten, medizinischen Stahl und/oder Titan bzw. den oben genannten Werkstoffen gefertigt sein.

Insbesondere kann der Stent-Brückenabschnitt ein Befestigungsmittel sein, während die Hülse ein Hülsen-Gegenstück ist. Umfasst die Hülse zusätzlich oder alternativ einen oder mehrere Vorsprünge, kann die Hülse auch ein Vorsprung-Gegenstück sein. In anderen Worten kann die Hülse gleichzeitig als Hülsen-Gegenstück und als Vorsprung-Gegenstück fungieren.

In diesem Fall ist der Stent-Brückenabschnitt das Befestigungsmittel und umfasst zusätzlich zumindest einen Kopplungsbereich, so dass die Hülse als Vorsprung-Gegenstück zumindest einen Vorsprung umfasst, und mittels des Vorsprungs mit dem Kopplungsbereich des Befestigungsmittels verbindbar ist, wobei der Vorsprung des Vorsprung-Gegenstücks in den Kopplungsbereich des Befestigungsmittels eingreift.

Der Kopplungsbereich des Befestigungsmittels kann beispielsweise als zylindrische und/oder konische Durchführung und/oder als Sackloch ausgebildet ist, wobei der Vorsprung des Vorsprung-Gegenstücks in den Kopplungsbereich des Befestigungsmittels eingreift, wenn das Befestigungsmittel mit dem Vorsprung-Gegenstück verbunden ist.

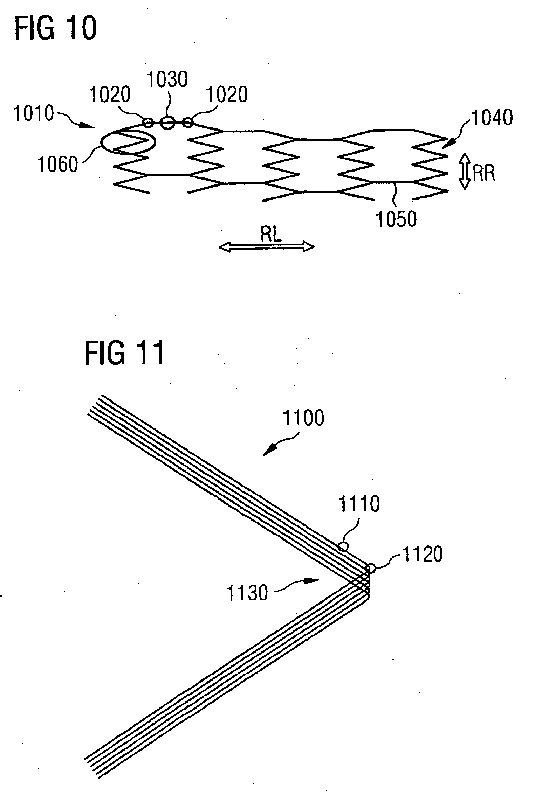

**Fig. 10** zeigt einen schematischen Ausschnitt einer Stentstruktur 1010, welche Verbindungselemente 1020 sowie eine zwischen den Verbindungselementen 1020 angeordnete Hülse 1030 umfaßt. Die Stentstruktur 1010 ist derart ausgebildet, daß der Stent in eine radiale Richtung RR expandiert werden kann, um Körpergewebe zu stützen und/oder mit Therapieträgern in Kontakt zu bringen. Weiterhin erstreckt sich die Stentstruktur 1010 in eine Längsrichtung RL, so daß mittels der Stentstruktur 1010 über einen bestimmten Längsbereich Körpergewebe gestützt werden kann. Insbesondere umfaßt die Stentstruktur 1010 Radialelemente 1040, welche dazu dienen Körpergewebe in der radialen Richtung RR zu stützen, wie beispielsweise einer verengten Vene. Zudem umfaßt die Stentstruktur 1010 längsverbindende Elemente 1050, welche dazu dienen, mehrere Radialelemente 1040 aneinander zu koppeln, so daß ebenfalls Körpergewebe, welches zwischen den Radialelementen 1040 liegt, gestützt werden kann, wenn der Stent im Körpergewebe positioniert ist.

Die Verbindungselemente 1020 können in einem Ausführungsbeispiel den Stent-Brückenabschnitte, wie zu Fig. 9 beschrieben, entsprechen. Ebenfalls kann die Hülse 1030 entsprechend einer Hülse, wie zu Fig. 9 beschrieben, entsprechen.

Entsprechend können die Verbindungselemente oder ein Teil der Verbindungselemente jeweils ein Substrat aus Nitiriol sein, welches ein oder mehrere Vorsprünge umfasst. Zusätzlich oder alternativ kann die Hülse oder ein Teil der Hülse ein Substrat aus Nitinol sein, welches ein oder mehrere Vorsprünge umfasst.

**Fig. 11** zeigt einen Ausschnitt 1100 einer Stentstruktur, welche insbesondere Multifilament-Geflechte 1110 umfaßt. Der Ausschnitt 1100 kann vorzugsweise einen Ausschnitt darstellen bzw. Teilbereich sein, wie in der Fig. 10 mit dem Bezugszeichen 1060 dargestellt.

Wie in Fig. 11 gezeigt, sind mehrere Multifilamentgeflechte 1110 im wesentlichen parallel zueinander angeordnet, und wobei eine Biegung 1130 vorgesehen ist.

Durch die Biegung 1130 kann ein Abknicken bzw. ein Richtungswechsel der Multifilamentgeflechte 1010 erzielt werden. Die Biegung 1130 kann derart ausgebildet sein, daß die Multifilamentgeflechte 1110 wie in Fig. 11 gezeigt, eine Pfeilspitze bzw. ein Hausdach beschreiben. In diesem Fall, können Steckenden 1120 vorgesehen sein, welche als Zwischenstücke zur Aufnahme der Multifilamentgeflechte 1110 dienen. Hierbei können die Multifilamentgeflechte 1110 und die Steckenden 1120 Kopplungsbereiche bzw. Vorsprünge aufweisen, mit welchen die Multifilamentgeflechte 1110 mit dem entsprechenden Steckende 1120 koppelbar sind. Diese Kopplungsbereiche bzw. Vorsprünge können entsprechend der hierin beschriebenen Vorsprünge bzw. Kopplungsbereiche ausgestaltet sein, wie beispielsweise zu Fig. 9. Insbesondere ist der Ausschnitt 1100 ein Ausschnitt eines geflochtenen Stents, bei dem die Steckenden 1120 zur Verbindung der Enden von jeweiligen Multifilamentgeflechten 1110 dienen.

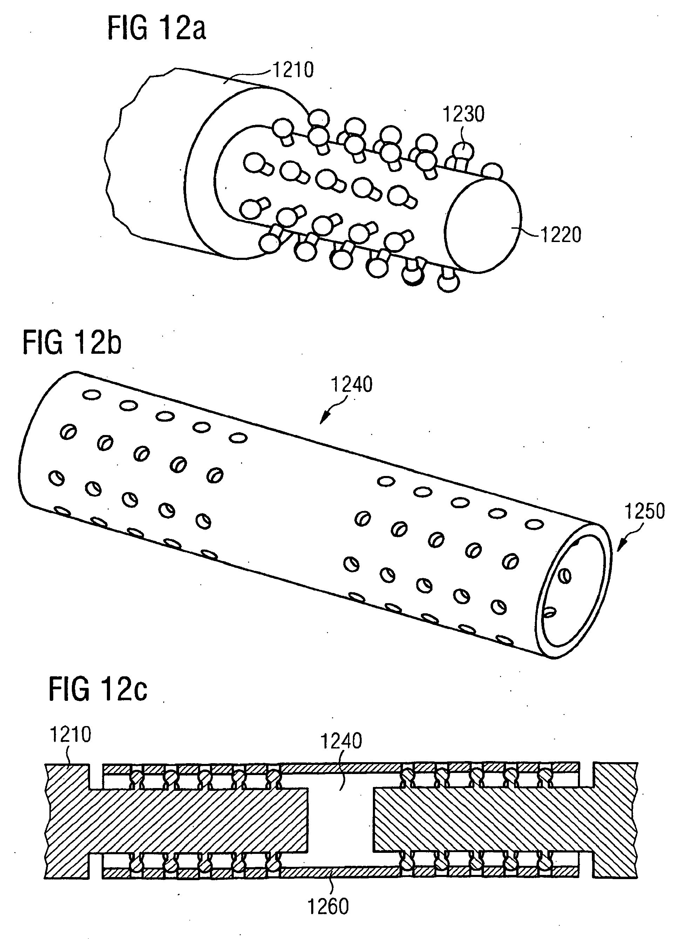

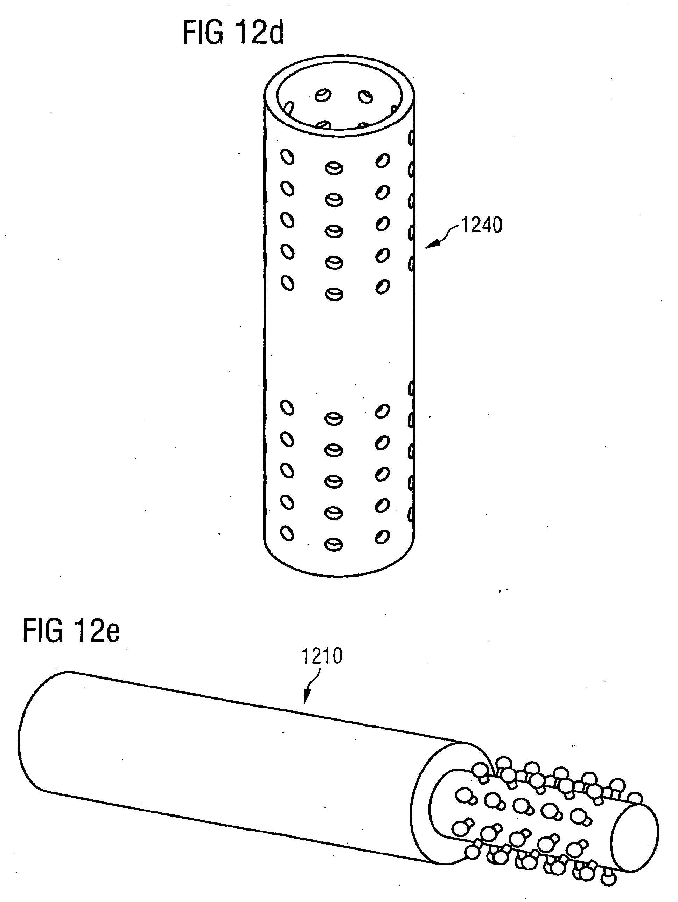

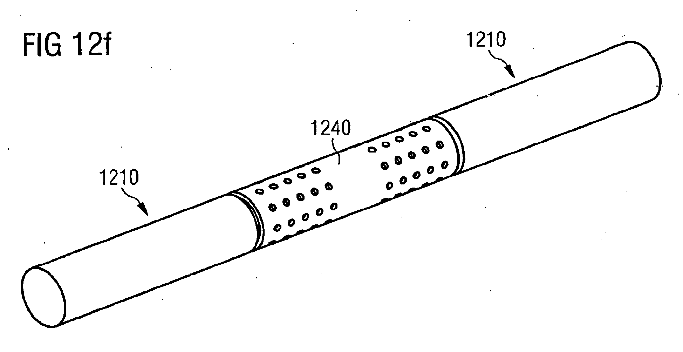

**Figs. 12a****,** **12e** **und** **12f** zeigen jeweils Abschnitte von Brückenabschnitten 1210 einer Stentstruktur, welche insbesondere Abschnitte bzw. Teile der bisher beschriebenen Stents/Stentstrukturen sein können. Die Brückenabschnitte 1210 umfassen insbesondere Fortsätze 1220. Die Fortsätze 1220 können an den Brückenabschnitten 1210 angeordnet sein. Vorzugsweise sind die Fortsätze 1220 an den Seitenflächen der Brückenabschnitte 1210 angeordnet. Alternativ kann ein Bereich eines Brückenabschitts 1210 als Fortsatz 1220 fungieren. Weiterhin sind an den Fortsätzen 1220 Vorsprünge 1230 angeordnet. Vorzugsweise sind die Vorsprünge 1230 radial um eine Mantelfläche der Fortsätze 1220 angeordnet.

Die Vorsprünge 1230 können insbesondere entsprechend der bereits vorhergehend beschriebenen Vorsprünge ausgebildet sein.

An die Fortsätze 1220 kann eine Hülse 1240 gekoppelt werden, welche entweder als bloße Hülse zum Verbinden oder als Medikamentenhülse (wie vorhergehend beschrieben) ausgebildet sein kann. In anderen Worten dienen die Fortsätze 1220 als Befestigungsmittel, um die Brückenabschnitte 1210 mit der Hülse zu verklippsen.

Die Hülse 1240 kann beispielsweise als Hohlzylinder ausgeformt sein, wobei in der Mantelfläche Kopplungsbereiche 1250 vorgesehen sind, welche zur Aufnahme der Vorsprünge 1230 bei einem Koppeln bzw. Einklippsen der Hülse 1240 und der Brückenabschnitte 1210 dienen. Alternativ zu einer hohlzylindrischen Ausführung der Hülse 1240 können auch Sacklöcher vorgesehen sein, welche die Aufnahme bzw. zumindest die teilweise Aufnahme der Fortsätze 1220 der Brückenabschnitte 1210 erlauben.

Insbesondere zeigt Fig. 12c eine Schnittansicht von Brückenabschnitten 1210 und einer Hülse 1240 in einem verklippsten Zustand. Wie mit den Bezugszeichen 1260 in der Fig. 12c dargestellt ist, kann eine Hülse 1240 beispielsweise eine Umhüllung bzw. Beschichtung aufweisen, welche Therapiemittel-/Medikamentengaben enthält und/oder aus röntgensichtbares Material umfasst.

Fig. 12f zeigt eine perspektivische Ansicht eines ähnlichen Stecksystems in einem zusammengesteckten Zustand, wie es in Fig. 9 beschrieben ist.

Zwar zeigen Fig. 9 und Fig. 12f jeweils Stent-Brückenabschnitte bzw. - Brückenteile, welche die Vorsprünge aufweisen und die jeweiligen Hülsen Kopplungsbereiche zur Aufnahme der Vorsprünge umfassen, jedoch ist es selbstverständlich denkbar, daß ein oder mehr Stent-Brückenabschnitte Kopplungsbereiche aufweisen und die entsprechenden Hülsen hingegen die Vorsprünge umfassen.

Auch kann die Ausführungsform, wie sie in den Figs. 12a bis 12f gezeigt werden, ebenfalls derart modifiziert sein, daß die Hülsen 1240 Biegungen oder Abknickungen aufweisen, so daß diese zum Beispiel als Steckenden, wie in Fig. 11 beschrieben, dienen können. Entsprechend kann auch die Hülse 1240 als lösbare Klippverbindung für geflochtene Drahtenden von Stents dienen.

In anderen Worten ist der Brückenabschnitt als das Befestigungsmittel mit einem Fortsatz ausgebildet. Der Fortsatz weist eine Vielzahl von hinterschnittenen Vorsprüngen auf, welche umfänglich an der Mantelfläche des Fortsatzes angeordnet sind, so dass die hinterschnittenen Vorsprünge in den Kopplungsbereich der Hülse, auch Fortsatz-Gegenstücks genannt, eingreifen, wenn der Fortsatz mit dem Fortsatz-Gegenstück verbunden ist, wobei das Fortsatz-Gegenstück als Hülse ausgebildet ist.

Umfasst die Hülse als Fortsatz-Gegenstück nicht nur Kopplungsbereiche sondern auch Vorsprünge (beispielsweise analog zu einem Fortsatz mit Vorsprüngen), dann ist die Hülse gleichzeitig ein Fortsatz-Gegenstück und ein Vorsprung-Gegenstück.

In anderen Worten zeigen die Figuren 12a und 12e einen Schaft, an dem sich pilzförmige Austragungen über den Umfang verteilt befinden und Figuren 12b und 12d zeigen ein Rohr, welches über dem Umfang Löcher hat. In diese Löcher passen die pitzförmigen Austragungen genau hinein. Wird der Schaft in das Rohr hineingesteckt, so verbiegen sich die pilzförmigen Austragungen elastisch. Im Rohr klicken diese in die Löcher ein und erzeugen einen festen Sitz. Die Folge ist, daß nun der Schaft mit dem Rohr verbunden ist. Um Schaft und Rohr zu lösen, ist lediglich ein Ziehen an dem Schaft notwendig. Daraufhin springen die Austragungen aus den Löchern aufgrund ihrer Elastizität heraus und die Trennung der Werkstücke ist möglich.

Entsprechend können die Brückenabschnitte oder nur die Fortsätze jeweils ein Substrat aus Nitinol sein, welches ein oder mehrere Vorsprünge umfasst. Zusätzlich oder alternativ kann die Hülse oder ein Teil der Hülse ein Substrat aus Nitinol sein, welches ein oder mehrere Vorsprünge umfasst.

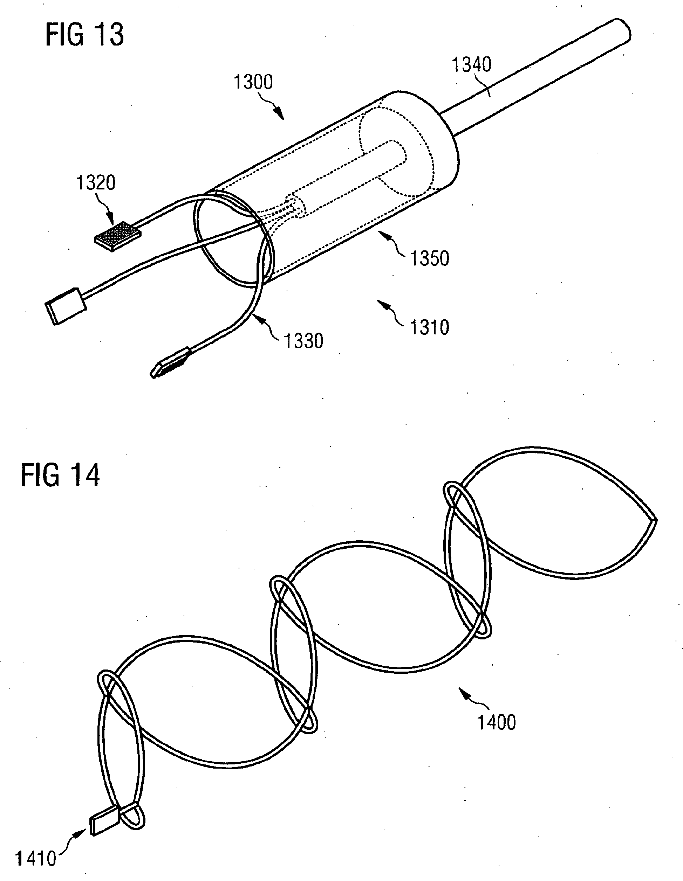

**Fig. 13** zeigt eine vereinfachte Darstellung eines Zuführsystem 1300, welches ein Verbindungselement 1310 und eine Teleskopstange 1340 umfaßt. Das Verbindungselement 1310 ist derart mit der Teleskopstange 1340 verbunden, daß das Verbindungselement 1310 mittels der Teleskopstange 1340 in eine Aufnahme 1350 hineingezogen oder aus der Aufnahme 1350 herausgeschoben werden kann. Die Aufnahme 1350 ist mit der Teleskopstange 1340 verbunden. Das Verbindungselement 1310 umfaßt insbesondere Verbindungsplatten 1320 und Drahtabschnitte 1330, wobei die Verbindungsplatten 1320 über Drahtabschnitte 1330 mit der Teleskopstange 1340 gekoppelt sind. An diese Verbindungsplatten 1320 kann beispielsweise ein Stent gekoppelt werden, der für neuroradiologische Untersuchungen oder sonstige medizinische Anwendungen ausgebildet ist. Ein entsprechender Stent bzw. ein entsprechendes neuroradiologisches Produkt ist beispielsweise in Fig. 14 gezeigt.

Entsprechend können die Verbindungsplatten oder ein Teil der Verbindungsplatten jeweils ein Substrat aus Nitinol sein, welches ein oder mehrere Vorsprünge umfasst.

**Fig. 14** zeigt ein medizinisches Produkt 1400, welches eine Verbindungsplatte 1410 umfaßt. Das medizinische Produkt 1400 kann beispielsweise ein Stent sein, der für neuroradiologische Zwecke einsetzbar ist.

Insbesondere können zum Zuführen, Positionieren oder Entfernen des medizinischen Produkts 1400 aus einem lebenden Körper die Verbindungsplatte 1410 mit zumindest einer Verbindungsplatte eines Zuführsystems gekoppelt bzw. verklippst werden, so daß das medizinische Produkt 1400 von einem Zuführsystem aufgenommen und gehalten wird bis eine gewünschte Positionierung oder Entfernung des medizinischen Produkts 1400 vollzogen ist. Hierzu kann beispielsweise das Zuführsystem 1300 verwendet werden.

Entsprechend kann die Verbindungsplatte oder ein Teil der Verbindungsplatte ein Substrat aus Nitinol sein, welches ein oder mehrere Vorsprünge umfasst.

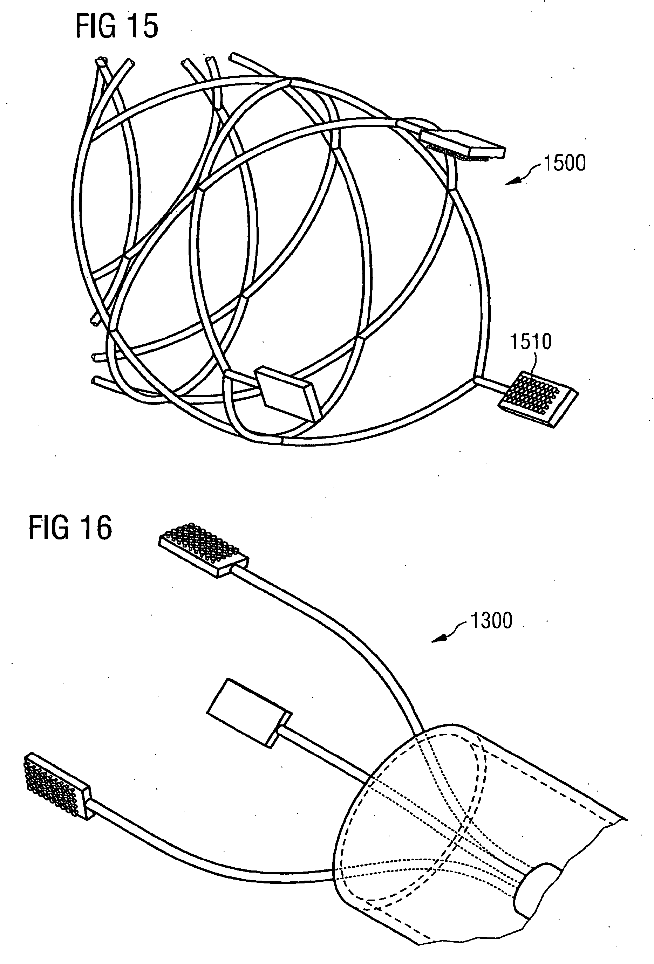

**Fig. 15** zeigt einen Ausschnitt eines mehrgliedrigen Verbindungselements 1500 mit Verbindungsplatten 1510. Insbesondere kann das Verbindungselement 1500 in einem Zuführsystem angewandt werden, wie dem Zuführsystem 1300, und mittels der Verbindungsplatten 1510 an einen Stent gekoppelt werden.

In anderen Worten handelt es sich um einen mehrgliedrigen Einzieher mit pilzförmigen Austragungen an den Enden. Das Gegenstück z.B. ein Stent hat an den Enden ebenso wie der Einzieher pilzförmige Austragungen. Berühren sich diese Austragungen, so verkeilen diese Austragungen sich miteinander.

**Fig. 16** zeigt einen vergrößerten Ausschnitt des Zuführsystems 1300, welches in Fig. 13 dargestellt ist.

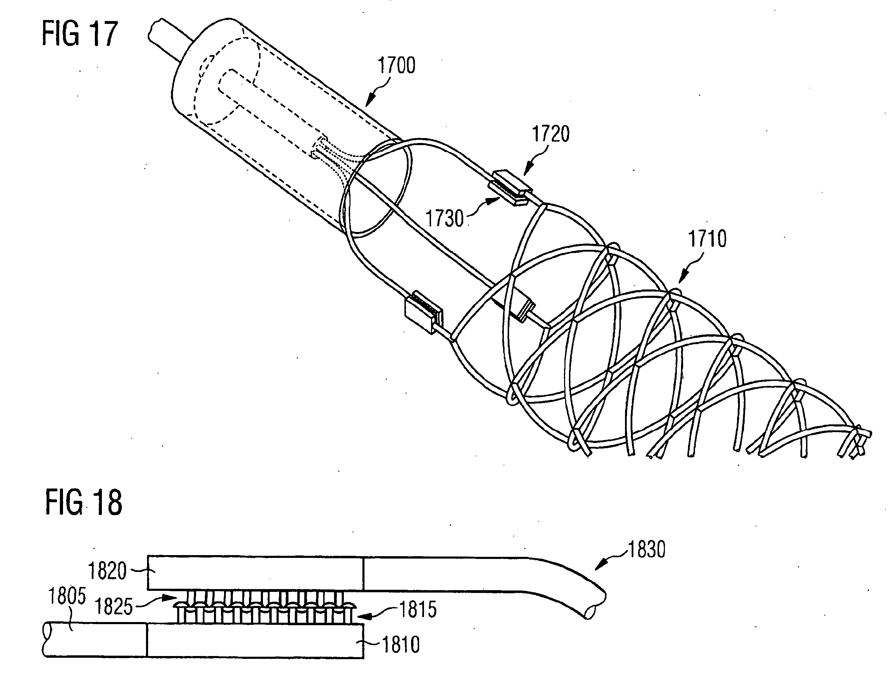

**Fig. 17** zeigt ein Zuführsystem 1700 mit einem daran angekoppelten Stent 1710, wobei das Zuführsystem 1700 und der Stent 1710 über Verbindungsplatten 1720 und 1730 aneinander gekoppelt sind.

**Fig. 18** zeigt eine Verbindungsplatte 1810, welche an einem Drahtabschnitt 1805 angeordnet ist, sowie eine damit verklippste Verbindungsplatte 1820, welche an einem Drahtabschnitt 1830 angeordnet ist. Wie aus der Figur 18 ersichtlich ist, umfassen die Verbindungsplatten jeweils mehrere Vorsprünge 1815, 1825 durch welche die Verbindungsplatten aneinander gekoppelt bzw. verklippst werden.

Beispielsweise kann eine der Verbindungsplatten zu einem Zuführsystem, wie beispielsweise dem Zuführsystem 1300, gehören und die andere Verbindungsplatte zu einem Stent. Sind die beiden Verbindungsplatten aneinander gekoppelt, kann das Zuführsystem bzw. Einzieher zurückgezogen werden, so daß sich aufgrund der Geometrie bzw. der Anordnung der Verbindungsplatten der verbundene bzw. angekoppelte Stent beim Einziehen krimpt.

Die Vorsprünge 1815 und 1825 können wie die in der Beschreibung vorangenannten Vorsprünge ausgebildet sein.

Entsprechend kann die Verbindungsplatte oder ein Teil der Verbindungsplatte ein Substrat aus Nitinol sein, welches ein oder mehrere Vorsprünge umfasst.

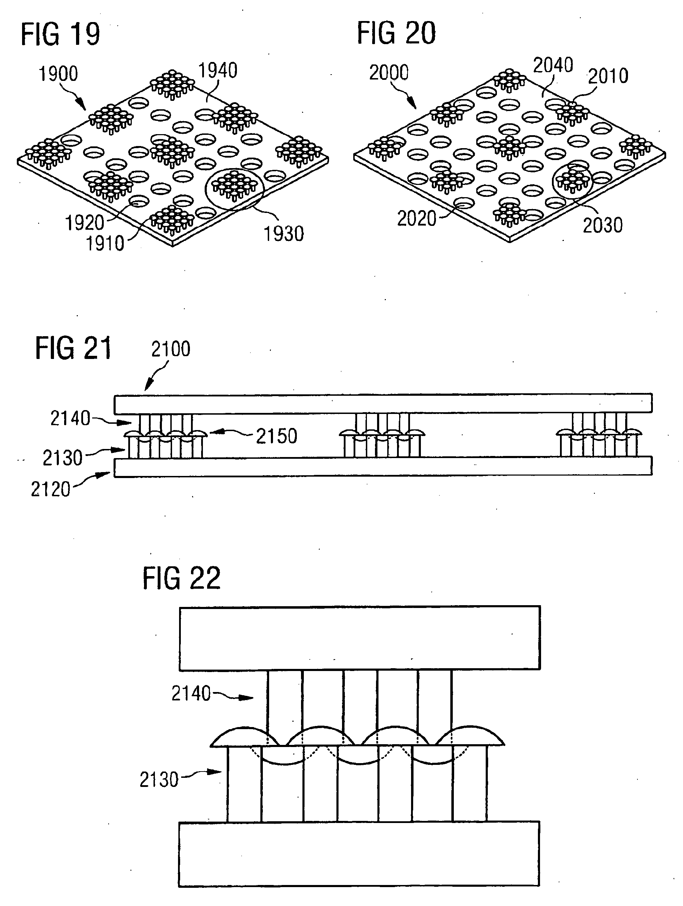

**Fig. 19** zeigt eine Verbindungsplatte 1900, welche Vorsprünge 1910 und Durchführungen 1920 umfaßt. Vorzugsweise ist die Verbindungsplatte 1900 in der Form eines Blechs, welches Abmasse im Bereich der Millimeter bis Nanometer aufweist. Insbesondere kann die Verbindungsplatte 1900 als Verbindungsplatte bei Stents oder Zuführsystemen verwendet werden, wie diese vorangehend beschrieben wurden (siehe insbesondere auch Figs. 13 bis 18).

Vorzugsweise sind die Durchführungen 1920 als Bohrungen ausgebildet, welche die gesamte Dicke einer Grundplatte 1940 der Verbindungsplatte 1900 durchdringen.

Die Vorsprünge 1910 sind vorzugsweise an einer Seitenfläche der Grundplatte 1940 angeordnet. In einer bevorzugten Ausführungsform werden die Vorsprünge 1910 gruppiert angeordnet, so daß mehrere Vorsprünge 1910 eine Insel 1930 bilden. Eine Insel 1930 kann zwei bis vier, vier bis acht, acht bis zwölf oder zwölf bis sechzehn Vorsprünge 1910 enthalten. In einer anderen Ausführungsform, können auch mehr als sechzehn Vorsprünge 1910 als Insel 1930 angeordnet werden. Wie in Fig. 19 gezeigt kann eine Insel 1930 eine im wesentlichen rechteckige Anordnung der Vorsprünge 1910 aufweisen. Alternativ kann aber auch vorgesehen werden, daß eine Insel durch eine andere geometrische Anordnung der Vorsprünge 1910 gebildet wird. Beispielsweise könnte eine Insel 1930 eine im wesentlichen kreisförmige Konfiguration von Vorsprüngen 1910 aufweisen oder eine ringförmige Formation von Vorsprüngen 1910, wobei ein oder mehrere Vorsprünge 1910 nebeneinander angeordnet sind, so daß die ringförmige Anordnung eine höhere Breite aufweist.

Die Vorsprünge 1910 können insbesondere jeweils pilzförmige Austragungen darstellen. Insbesondere können die Vorsprünge 1910 sämtliche vorhergehend beschriebenen Formen und Eigenschaften umfassen. Wie aus der Fig. 19 ersichtlich ist, stehen die Vorsprünge 1910 von der Grundplatte 1940 ab bzw. ragen hervor. Insbesondere ist die Verbindungsplatte 1900 dazu geeignet mit einer weiteren Verbindungsplatte gekoppelt bzw. verklippst zu werden. Entsprechend kann die Verbindungsplatte oder ein Teil der Verbindungsplatte ein Substrat aus Nitinol sein, welches ein oder mehrere Vorsprünge umfasst.

**Fig. 20** zeigt eine Verbindungsplatte 2000 mit Vorsprüngen 2010, Durchführungen 2020 und einer Grundplatte 2040. Die Vorsprünge 2010 sind in kleineren Gruppen gruppiert und bilden mehrere Inseln 2030. Eine Gruppe 2030 kann wie dargestellt neun Vorsprünge 2010 aufweisen, welche im wesentlichen rechteckig angeordnet sind. Alternativ können auch Inseln mit drei bis sechs Vorsprüngen sechs bis neun Vorsprüngen neun bis zwölf Vorsprüngen oder mehr Vorsprüngen gebildet werden.

Die Vorsprünge 2010 können insbesondere eine Form ausbilden, wie es vorangehend bereits für verschiedene Vorsprünge beschrieben wurde.

Insbesondere können in die Durchführungen 1920 bzw. 2020 Therapiematerial eingebracht werden. Beispielsweise könnte röntgensichtbares Material, wie Gold, in eine oder mehrere Durchführungen eingesetzt werden, so daß die Position der Verbindungsplatten, welche an einem Stent oder Zuführsystem angeordnet sein können, nachvollziehbar ist, wenn diese in zu untersuchende oder zu behandelnde Körpergewebe eingeführt oder herausgenommen werden. Entsprechend kann die Verbindungsplatte oder ein Teil der Verbindungsplatte ein Substrat aus Nitinol sein, welches ein oder mehrere Vorsprünge umfasst.

**Fig. 21** zeigt eine Verbindungsplatte 2100 sowie eine weitere Verbindungsplatte 2120, welche über Vorsprünge 2130 und 2140 miteinander verklippst sind. Beispielsweise können die Verbindungsplatten 2100 und 2120 den vorhergehend beschriebenen Verbindungsplatten 1900 entsprechen. Wie aus Fig. 21 ersichtlich ist, können die Verbindungsplatten 2100 und 2120 durch die Vorsprünge 2130 und 2140 derart aufeinander gesteckt werden, daß sich die Vorsprünge ineinander verhaken, sobald die Verbindungsplatten zusammengedrückt bzw. aneinander gekoppelt werden. Hierzu sind die Vorsprünge 2130 und 2140 versetzt zueinander angeordnet. Bei einem Zusammendrücken der Verbindungsplatten 2100 und 2120, werden die Vorsprünge 2130 elastisch von den Vorsprüngen 2140 beiseite gedrückt, so daß die Vorsprünge 2140 den ansonsten verengten Bereich 2150 in einen aufgeweiteten Bereich elastisch verformen, so daß die Vorsprünge 2140 vorbeigleiten können und sobald der aufgeweitete Bereich passiert wurde, die Vorsprünge 2130 in ihre ursprüngliche Position zurückkehren, so daß dann eine Verklippsung bzw. Einhakung der Vorsprünge 2130 und 2140 ineinander erfolgt ist.

Insbesondere bilden die Verbindungsplatten 2100 und 2120 ein Befestigungsmittel und ein Gegenstück. Beispielsweise kann die Verbindungsplatte 2100 als Befestigungsmittel angesehen werden und die Verbindungsplatte 2120 als entsprechendes Gegenstück oder umgekehrt. Insbesondere kann eine Verbindungsplatte gleichzeitig Befestigungsmittel und Gegenstück sein. Beispielsweise ist die Verbindungsplatte als Befestigungsmittel ausgebildet und umfasst eine Vielzahl von Vorsprüngen 2140, während die Verbindungsplatte 2120 ein Verbindungsplatten-Gegenstück darstellt, welches ebenfalls eine Vielzahl von Vorsprüngen 2130 aufweist, wobei die Vorsprünge 2130, 2140 des Befestigungsmittels 2100 und des Verbindungsplatten-Gegenstücks 2120 derart zueinander angeordnet sind, dass das Befestigungsmittel 2100 mit dem Verbindungsplatten-Gegenstück 2120 durch Verhaken der Vorsprünge 2130; 2140 ineinander verbindbar ist.

**Fig. 22** zeigt einen vergrößerten Ausschnitt der Fig. 21, wobei hierbei insbesondere Vorsprünge 2140 und 2130 gezeigt werden, wobei die ineinander versetzte Anordnung der Vorsprünge 2130 und 2140 ersichtlich ist.

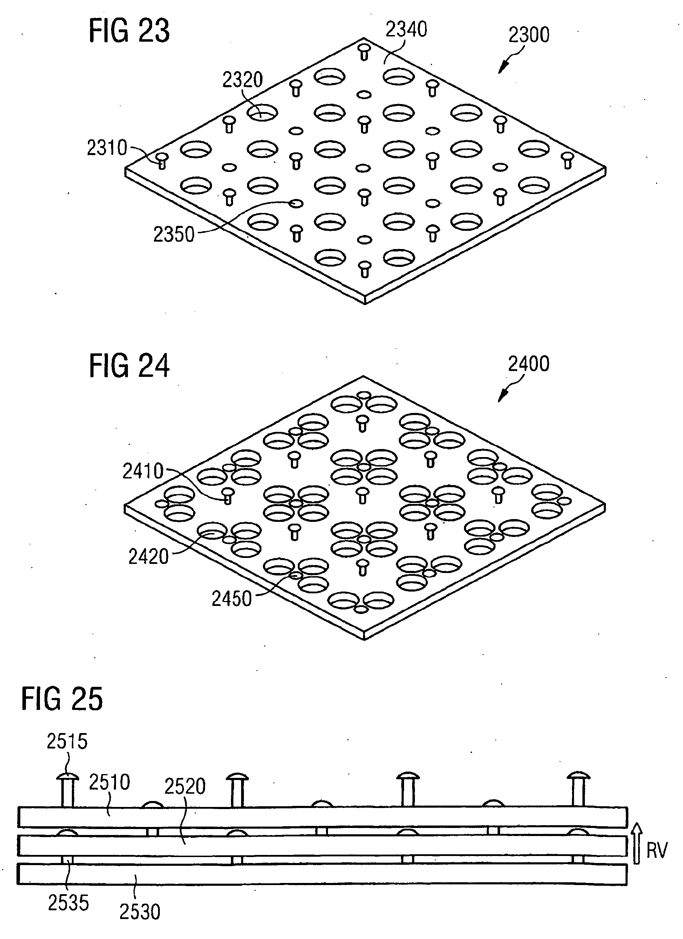

**Fig. 23** zeigt eine Verbindungsplatte 2300 mit Vorsprüngen 2310, Durchführungen 2320 und einer Grundplatte 2340, wobei zusätzlich Kopplungsbereiche 2350 in bzw. an der Grundplatte 2340 angeordnet sind. Im Gegensatz zu den vorherigen Ausführungsformen sind nun die Vorsprünge 2310 einzeln bzw. umgruppiert angeordnet. Die Kopplungsbereiche 2350 sind insbesondere derart ausgebildet, daß diese Vorsprünge anderer Verbindungsplatten aufnehmen können.

Die Vorsprünge 2310 können derart angeordnet sein, daß die Verbindungsplatte 2300 mit einer Verbindungsplatte wie der Verbindungsplatte 1900 und/oder der Verbindungsplatte 2010 oder einer Verbindungsplatte wie in den Figs. 15 bis 18 gezeigt koppelbar ist.

**Fig. 24** zeigt eine Verbindungsplatte 2400 mit Vorsprüngen 2410, Durchführungen 2420, Durchführungen 2440 und Kopplungsbereichen 2450. Auch in Fig. 24 sind die Vorsprünge 2410 einzeln angeordnet. Zwar bilden auch hier die Vorsprünge 2410 eine im wesentlichen rechteckige Anordnung aus, jedoch kann auch eine kreisrunde oder sonstige geometrische Anordnung vorgesehen sein. Insbesondere ist es denkbar die Verbindungsplatte 2400 mit einer der vorangehend beschriebenen Verbindungsplatten zu koppeln bzw. zu verklippsen. In einer bevorzugten Ausführungsform, kann die Verbindungsplatte 2400 mit der Verbindungsplatte 2300 verklippst werden.

**Fig. 25** zeigt drei ineinander verklippste Verbindungsplatten 2510, 2520 und 2530, welche jeweils Vorsprünge 2515, 2525 und 2535 aufweisen. In der in Fig. 25 dargestellten Ausführungsform, sind die Platten derart aneinander angeordnet, daß die Vorsprünge 2515, 2525 und 2535 jeweils mit ihren Enden in die gleiche Richtung RV ragen. Entsprechend dieser Konfiguration ist es möglich, an der Verbindungsplatte 2510 eine weitere Verbindungsplatte anzuordnen. Eine weitere Verbindungsplatte, die an der Verbindungsplatte 2510 angeordnet werden kann, könnte beispielsweise die Verbindungsplatte 1900, die Verbindungsplatte 2000 oder die Verbindungsplatte 2400 sein.

In der in Fig. 25 gezeigten Ausführungsform, können die Verbindungsplatten 2510 und 2530 beispielsweise der Verbindungsplatte 2300 entsprechen und die Verbindungsplatte 2520 der Verbindungsplatte 2400.

In einer anderen Konfiguration könnte beispielsweise die Verbindungsplatte 2410 weggelassen werden und die Verbindungsplatte 2520 derart an der Verbindungsplatte 2530 angeordnet sein, daß ihre Vorsprünge 2425 in die Verbindungsplatte 2530 eingreifen. In dieser Ausführungsform würde eine doppelte Verklippsung stattfinden, da die Vorsprünge 2525 und 2535 jeweils in die Verbindungsplatte der anderen Verbindungsplatte eingreifen.

In anderen Worten können eine oder mehrere der Verbindungsplatten 2510, 2520, 2530 als Befestigungsmittel und/oder als Gegenstück fungieren. Jede der Verbindungsplatten 2510, 2520, 2530 weist Kopplungsbereiche sowie Vorsprünge auf, so dass jede der Verbindungsplatten 2510, 2520, 2530 gleichzeitig oder alternativ als Befestigungsmittel oder als Gegenstück eingesetzt werden kann.

In einem Beispiel ist die Verbindungsplatte 2520 ein Befestigungsmittel, welches zumindest einen Kopplungsbereich umfasst, und die Verbindungsplatte 2530 ein Vorsprung-Gegenstück, welches zumindest einen Vorsprung 2535 umfasst, mittels dieses Vorsprungs 2535 ist das Vorsprung-Gegenstück 2530 mit dem Kopplungsbereich des Befestigungsmittels 2520 verbindbar ist, wobei der Vorsprung 2535 des Vorsprung-Gegenstücks 2530 in den Kopplungsbereich des Befestigungsmittels 2520 eingreift.

Der Kopplungsbereich der Verbindungsplatten 2510, 2520, 2530 (Befestigungsmittel) kann als im wesentlichen zylindrische und/oder im wesentlichen konische Durchführung und/oder als Sackloch ausgebildet sein.

Insbesondere kann eine Verbindungsplatte, welche als Gegenstück fungiert, gleichzeitig oder alternativ ein Vorsprung-Gegenstück bzw. ein Verbindungsplatten-Gegenstück sein.

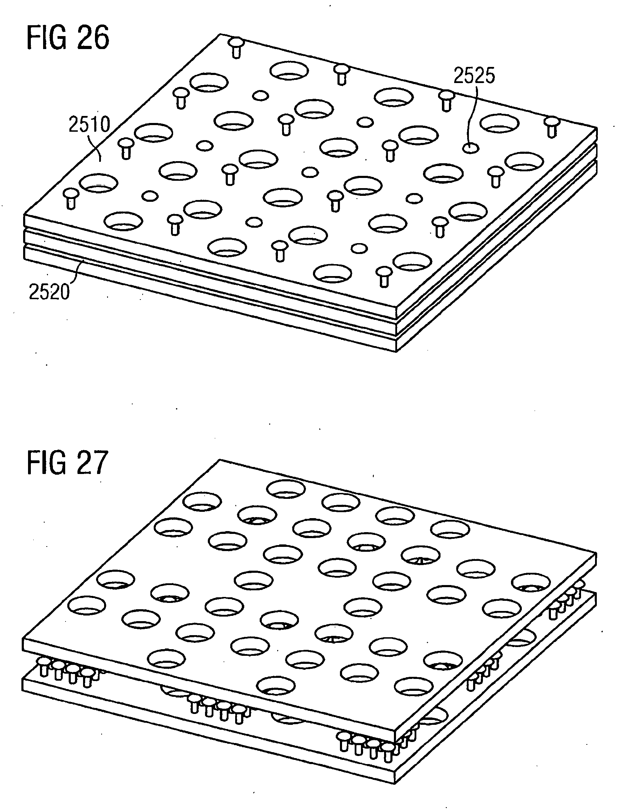

**Fig. 26** zeigt eine perspektivische Ansicht der Fig. 25. Hierbei ist erkennbar, daß die Vorsprünge 2525 der Verbindungsplatte 2520 in Kopplungsbereiche der Verbindungsplatte 2510 eingreifen.

**Fig. 27** zeigt eine perspektivische Ansicht von verklippsten Verbindungsplatten, wobei diese Verbindungsplatten den vorhergehend beschriebenen Verbindungsplatten entsprechen kann. Insbesondere können die Vorsprünge sowie die Kopplungsbereiche der Verbindungsplatten derart ausgebildet sind, daß diese Formen und Eigenschaften der in den anderen Ausführungsbeispielen entsprechen. Insbesondere können die Vorsprünge und Kopplungsbereiche der Verbindungsplatten den Vorsprüngen und Kopplungsbereiche der Stent-Brückenabschnitte und Hülsen entsprechen. Entsprechend kann jede Verbindungsplatte oder ein Teil einer jeden Verbindungsplatte ein Substrat aus Nitinol sein, welches ein oder mehrere Vorsprünge umfasst.

Insbesondere können die beschriebenen Stentstrukturen oder Abschnitte von Stentstrukturen bzw. Stent-Stege oder Stent-Brückenabschnitte, Fortsätze sowie Verbindungsplatten Befestigungsmittel sein. Weiterhin können die beschriebenen Medikamententräger, Hülsen sowie Verbindungsplatten Gegenstücke sein.

Insbesondere können die beschriebenen Stentstrukturen oder Abschnitte von Stentstrukturen bzw. Stent-Stege oder Stent-Brückenabschnitte, Fortsätze sowie Verbindungsplatten jeweils ein vorangehend beschriebenes Substrat umfassen, vorzugsweise aus Nitinol für biokompatible und/oder hochfeste Mikro-Verbindungen umfassen. Alternativ oder zusätzlich können die beschriebenen Medikamententräger, Hülsen sowie Verbindungsplatten ebenfalls ein vorangehend beschriebenes Substrat umfassen, vorzugsweise aus Nitinol für biokompatible und/oder hochfeste Mikro-Verbindungen.

Die Vorteile der Befestigung mittels elastischer Vorsprünge liegen vor allem in den superelastischen Werkstoffeigenschaften von Nitinol, das sowohl Verbiegungen als auch Formänderungen erlaubt, ohne sich plastisch zu verformen. Auf vorteilhafte Weise wird die Vielzahl der Vorsprünge 20, 40 durch PECM (precise electrochemical machining) hergestellt, und zwar mit Hilfe des in Fig. 3 dargestellten Elektrodenfeldes 30. Weiter bevorzugt wird hier ein sogenanntes 2,5 D Array-Strukturverfahren verwendet, bei dem die dritte Dimension keine Freiformherstellung erlaubt. Dabei wird eine Senkung in das Material, d.h. in einer Z-Achse mit einem 2D-Werkzeug durchgeführt, ohne daß hierzu eine vorgegebene 3D-Bearbeitungsachse erforderlich wäre.

Die Erfindung ist nicht auf die hier beschriebenen Anwendungen beschränkt, sondern kann bei vielzähligen anderen Anwendungen verwendet werden. Darüber hinaus ist die Form der Vorsprünge 20 nicht auf die hier gezeigte Pilzform beschränkt, sondern es kann jede andere Art einer hinterschnittenen Form durch das erfindungsgemäße Verfahren erzeugt werden. Das erfindungsgemäße Befestigungsmittel ist insbesondere temperaturunempfindlich, weil es aus Metall hergestellt ist. Darüber hinaus wird eine höhere Genauigkeit beispielsweise in der Halbleiterindustrie gegenüber Klebeverfahren nach dem Stand der Technik erzielt.

Es können auch Polymere, die beispielsweise durch MicroMolding hergestellt werden, oder andere metallische, superelastische Werkstoffe oder elastische Federkonstruktionen verwendet werden. Als Herstellungsmethode eignen sich auch Mikrospritzguss und Lasersintern mit zusätzlicher Kaltverformung beispielsweise durch Strahl- oder Peening-Prozesse.

### Bezugszeichenliste

10 Substrat
20, 40 Vorsprung
22 Abschnitt großen Durchmessers
24 Abschnitt kleinen Durchmessers
26 Rand
30 Elektrodenfeld
32 Elektrode
42 Abschnitt großen Durchmessers
44 Abschnitt kleinen Durchmessers
100 Stentstruktur
120 Medikament
200 makroskopische Erhöhung der Oberfläche
300 Nanooberfläche
400 Oberflächenabwandlung
950, 1230, 1815, 1825, 1910, 2010, 2130, 2140, 2310, 2410, 2515, 2525, 2535 Vorsprung
910, 930,1210 Brückenabschnitt
920, 1240 Hülse
940, 1250, 1920, 2020, 2320, 2420 Kopplungsbereich
1320, 1410, 1510, 1720, 1810, 1820, 1900, 2000, 2100, 2120, 2300, 2400, 2510, 2520, 2530 Verbindungsplatte
1940, 2040, 2340 Grundplatte
1920, 2020, 2420 Durchführungen
2820, 2920, 3020 Multi-Lumen-Hülsen

## Patentansprüche

1. Stent (100) mit einem Medikamententräger zum Implantieren in den menschlichen Körper mit einem Substrat (10) aus Nitinol für biokompatible und/oder hochfeste Mikro-Verbindungen, wobei eine Vielzahl von hinterschnittenen Vorsprüngen (20) von dem Substrat (10) hervorragt, um ein Verhaken der hinterschnittenen Vorsprünge (20) mit dem Medikamententräger als entsprechenden Gegenstück zu ermöglichen, so dass der Stent lösbar mit dem Medikamententräger verbunden ist.

2. Stent nach Anspruch 1, das zumindest ein Paar Substrate (10) mit einer Vielzahl von hinterschnittenen Vorsprüngen (20, 40) aufweist, die so gestaltet sind, dass die beiden Substrate lösbar miteinander verbindbar sind, indem die Vorsprünge (20) eines Substrats mit den Vorsprüngen (40) des anderen Substrats miteinander verhaken.

3. Stent nach einem der vorherigen Ansprüche, wobei zumindest eine Gruppe der Vorsprünge (20, 40) wenigstens teilweise einen im wesentlichen kreisförmigen Querschnitt aufweisen und/oder zumindest eine Gruppe der Vorsprünge (20, 40) im wesentlichen pilzförmig geformt ist.

4. Stent nach einem der vorherigen Ansprüche, wobei zumindest eine Gruppe der Vorsprünge (20, 40) zumindest einen Abschnitt kleinen Querschnitts (24, 44) und zumindest einen Abschnitt großen Querschnitts (22, 42) aufweisen, wobei der Abschnitt kleinen Querschnitts (24, 44) zwischen dem Substrat (10) und dem Abschnitt großen Querschnitts (22, 42) angeordnet ist.

5. Stent nach einem der vorherigen Ansprüche, wobei ein Rand (26) des Abschnitts großen Querschnitts (22) abgerundet und/oder angeschrägt/angefast und/oder konisch ist.

6. Stent nach einem der vorherigen Ansprüche, wobei die Vorsprünge (20, 40) in einem Abstand von 50 bis 2000 µm, vorzugsweise in einem Abstand von 100 bis 300 µm voneinander an dem Substrat (10) angeordnet sind.

7. Stent nach einem der vorherigen Ansprüche, wobei der Abschnitt großen Querschnitts (22, 42) einen Durchmesser von 20 bis 300 µm, vorzugsweise von 50 bis 200 µm hat und/oder der Abschnitt kleinen Querschnitts (24, 44) einen Durchmesser von 1 bis 300 µm, vorzugsweise von 40 bis 150 µm hat.

8. Befestigungsmittel (100; 910; 1210; 2100; 2120; 2520) mit einem Medikamententräger und einem Substrat aus Nitinol für biokompatible und/oder hochfeste Mikro-Verbindungen, wobei zumindest ein hinterschnittener Vorsprung (20) von dem Substrat hervorragt, um ein Verhaken des hinterschnittenen Vorsprungs mit dem Medikamententräger als entsprechenden Gegenstück zu ermöglichen, so dass das Befestigungsmittel lösbar mit dem Gegenstück verbunden ist.

9. Befestigungsmittel (2520) nach Anspruch 8, wobei das Befestigungsmittel (2520) zusätzlich zumindest einen Kopplungsbereich umfasst, so dass ein Vorsprung-Gegenstück (2530), welches ebenfalls zumindest einen Vorsprung (2535) umfasst, mittels des Vorsprungs (2535) mit dem Kopplungsbereich des Befestigungsmittels (2520) verbindbar ist, wobei der Vorsprung (2535) des Vorsprung-Gegenstücks (2530) in den Kopplungsbereich des Befestigungsmittels (2520) eingreift.

10. Befestigungsmittel (2520) nach Anspruch 9, wobei der Kopplungsbereich des Befestigungsmittels (2520) als zylindrische oder konische Durchführung oder als Sackloch ausgebildet ist, wobei der Vorsprung (2535) des Vorsprung-Gegenstücks (2530) in den Kopplungsbereich des Befestigungsmittels (2520) eingreift, wenn das Befestigungsmittel (2520) mit dem Vorsprung-Gegenstück (2530) verbunden ist.

11. Befestigungsmittel (910) nach einem der Ansprüche 8 bis 10, wobei das Befestigungsmittel (910) als Brückenabschnitt ausgebildet ist, so dass der hinterschnittene Vorsprung (950) in den Kopplungsbereich (940) eines Hülsen-Gegenstücks (920) eingreift, wenn der Brückenabschnitt mit dem Hülsen-Gegenstück (920) verbunden ist, wobei das Hülsen-Gegenstück (920) als Medikamentenhülse ausgebildet ist.

12. Befestigungsmittel (1210) nach einem der Ansprüche 8 bis 10, wobei das Befestigungsmittel (1210) einen Fortsatz (1220) umfasst, wobei der Fortsatz (1220) eine Vielzahl von hinterschnittenen Vorsprüngen (1230) aufweist, welche umfänglich an der Mantelfläche des Fortsatzes (1220) angeordnet sind, so dass die hinterschnittenen Vorsprünge (1230) in den Kopplungsbereich (1250) eines Fortsatz-Gegenstücks (1240) eingreifen, wenn der Fortsatz (1220) mit dem Fortsatz-Gegenstück (1240) verbunden ist, wobei das Fortsatz-Gegenstück (1240) als Medikamentenhülse ausgebildet ist.

13. Verfahren zum Herstellen eines Befestigungsmittels gemäß der Ansprüche 8 bis 12 mit den Schritten:
Bereitstellen eines Substrats (10) aus Nitinol;
elektrochemisches Bearbeiten einer Oberfläche des Substrats (10) zum Erzeugen einer Vielzahl von hinterschnittenen Vorsprüngen (20) an der Oberfläche des Substrats (10); und
Verbinden des Befestigungsmittels mit einem Medikamententräger als Gegenstück.

14. Verfahren nach Anspruch 13, wobei die elektrochemische Bearbeitung mit Hilfe eines Elektrodenfelds (30) erfolgt, wobei Elektroden (32) des Elektrodenfelds (30) isolierte Seitenflanken aufweisen.

## Claims

1. A stent (100) with a medicament carrier for implantation in the human body, including a substrate (10) made of nitinol for biocompatible and/or high-strength micro-connections, wherein a plurality of undercut projections (20) project from the substrate (10) for enabling engagement of the undercut projections (20) with the medicament carrier as a corresponding counter piece such that the stent is detachably connected to the medicament carrier.

2. The stent according to claim 1, including at least a pair of substrates (10) having a plurality of undercut projections (20, 40) formed such that the two substrates are detachably connectable to one another by means of the projections (20) of one substrate engaging with the projections (40) of the other substrate.

3. The stent according to any one of the preceding claims, wherein at least one group of the projections (20, 40) at least partially has a substantially circular cross-section and/or at least one group of the projections (20, 40) is formed substantially in a mushroom-shape.

4. The stent according to any one of the preceding claims, wherein at least one group of the projections (20, 40) has at least one small cross-section portion (24, 44) and at least one large cross-section portion (22, 42), with the small cross-section portion being arranged between the substrate (10) and the large cross-section portion (22, 42).

5. The stent according to any one of the preceding claims, wherein an edge of the large cross-section portion (22) is rounded and/or tapered/bevelled and/or conical.

6. The stent according to any one of the preceding claims, wherein the projections (20, 40) are arranged on the substrate (10) at a mutual distance of 50 to 2000 µm, preferably at a distance of 100 to 300 µm,.

7. The stent according to any one of the preceding claims, wherein the large-cross-section portion (22, 42) has a diameter of 20 to 300 µm, preferably of 50 to 200 µm, and/or the small-cross-section portion (24, 44) has a diameter of 1 to 300 µm, preferably of 40 to 150 µm.

8. A fastening means (100; 910; 1210; 2100; 2120; 2520) including a medicament carrier and a nitinol substrate for biocompatible and/or high-strength micro-connections, wherein at least one undercut projection (20) projects from the substrate for enabling engagement of the undercut projection with the medicament carrier as a corresponding counter piece such that the fastening means is detachably connected to the counter piece.

9. The fastening means (2520) according to claim 8, wherein the fasting means (2520) additionally includes at least a coupling area such that a projection counter piece (2530) also including a projection (2535) is connectable to the coupling area of the fastening means via the projection (2535), with the projection (2535) of the projection counter piece engaging with the coupling area of the fastening means (2520).

10. The fastening means (2520) according claim 9, wherein the coupling area of the fastening means (2520) is formed as a cylindrical or conical feedthrough or as a blind hole, with the projection (2535) of the projection counter piece (2530) engaging with the coupling area of the fastening means (2520) when the fastening means (2520) is connected to the projection counter piece (2530).

11. The fastening means (910) according to any one of claims 8 to 10, wherein the fastening means is formed as a bridge portion such that the undercut projection (950) engages with the coupling area (940) of a cartridge counter piece (920) when the bridge portion is connected to the cartridge counter piece (920), wherein the cartridge counter piece (920) is formed as a medicament cartridge.

12. The fastening means (1210) according to any one of claims 8 to 10, wherein the fastening means (1210) comprises an extension (1220), wherein the extension (1220) has a plurality of undercut projections (1230) arranged circumferentially on the lateral surface of the extension (1220) such that the undercut projections (1230) engage with the coupling area (1250) of an extension counter piece (1240) when the extension (1220) is connected to the extension counter piece (1240), with the extension counter piece being formed as a medicament cartridge.

13. A method of producing a fastening means according to claims 8 to 12, comprising the steps of:
providing a substrate (10) made of nitinol;
electrochemical treatment of a surface of the substrate (10) for generating a plurality of undercut projections (20) on the surface of the substrate (10); and
connecting the fastening means to a medicament carrier as a counter piece.

14. The method according to claim 13, wherein the electrochemical treatment is performed by means of an electrode array (30), wherein electrodes (32) of the electrode array (30) have insulated side faces.

## Revendications

1. Endoprothèse vasculaire (100) avec un support de médicament pour l'implantation dans le corps humain avec un substrat (10) en nitinol pour des microconnexions biocompatibles et/ou hautement solides, dans laquelle une pluralité de saillies contre-dépouillées (20) dépasse du substrat (10) pour permettre un accrochage des saillies contre-dépouillées (20) avec le support de médicament en tant que pendant correspondant de sorte que l'endoprothèse vasculaire est reliée de manière amovible au support de médicament.

2. Endoprothèse vasculaire selon la revendication 1, qui présente au moins une paire de substrats (10) avec une pluralité de saillies contre-dépouillées (20, 40) qui sont configurées de sorte que les deux substrats peuvent être reliés de manière amovible l'un à l'autre en ce que les saillies (20) d'un substrat s'accrochent ensemble avec les saillies (40) de l'autre substrat.

3. Endoprothèse vasculaire selon une des revendications précédentes, dans laquelle au moins un groupe de saillies (20, 40) présente au moins partiellement une section transversale essentiellement circulaire et/ou au moins un groupe de saillies (20, 40) est moulé essentiellement en forme de champignon.

4. Endoprothèse vasculaire selon une des revendications précédentes, dans laquelle au moins un groupe de saillies (20, 40) présente au moins une section de petite section transversale (24, 44) et au moins une section de grande section transversale (22, 42), dans laquelle la section de petite section transversale (24, 44) est disposée entre le substrat (10) et la section de grande section transversale (22, 42).

5. Endoprothèse vasculaire selon une des revendications précédentes, dans laquelle un bord (26) de la section de grande section transversale (22) est arrondi et/ou biseauté/chanfreiné et/ou conique.

6. Endoprothèse vasculaire selon une des revendications précédentes, dans laquelle les saillies (20, 40) sont disposées à un écart de 50 à 2000 µm, de préférence à un écart de 100 à 300 µm les unes des autres sur le substrat (10).

7. Endoprothèse vasculaire selon une des revendications précédentes, dans laquelle la section de grand section transversale (22, 42) a un diamètre de 20 à 300 µm, de préférence de 50 à 200 µm et/ou la section de petit section transversale (24, 44) a un diamètre de 1 à 300 µm, de préférence de 40 à 150 µm.

8. Moyen de fixation (100 ; 910 ; 1210 ; 2100 ; 2120 ; 2520) avec un support de médicament et un substrat en nitinol pour des microconnexions biocompatibles et/ou hautement solides, dans lequel au moins une saillie contre-dépouillée (20) dépasse du substrat pour permettre un accrochage de la saillie contre-dépouillée avec le support de médicament en tant que pendant correspondant de sorte que le moyen de fixation est relié de manière amovible au pendant.

9. Moyen de fixation (2520) selon la revendication 8, dans lequel le moyen de fixation (2520) comprend en outre au moins une région d'accouplement de sorte qu'un pendant de saillie (2530) qui comprend également au moins une saillie (2535) peut être relié au moyen de la saillie (2535) à la région d'accouplement du moyen de fixation (2520), dans lequel la saillie (2535) du pendant de saillie (2530) s'engrène dans la région d'accouplement du moyen de fixation (2520).

10. Moyen de fixation (2520) selon la revendication 9, dans lequel la région d'accouplement du moyen de fixation (2502) est réalisée en tant que passage cylindrique ou conique ou en tant que trou borgne, dans lequel la saillie (2535) du pendant de saillie (2530) s'engrène dans la région d'accouplement du moyen de fixation (2520) lorsque le moyen de fixation (2520) est relié au pendant de saillie (2530).

11. Moyen de fixation (910) selon une des revendications 8 à 10, dans lequel le moyen de fixation (910) est réalisé en tant que section de pont de sorte que la saillie contre-dépouillée (950) s'engrène dans la région d'accouplement (940) d'un pendant de manchon (920) lorsque la section de pont est reliée au pendant de manchon (920), dans lequel le pendant de manchon (920) est réalisé en tant que manchon de médicament.

12. Moyen de fixation (1210) selon une des revendications 8 à 10, dans lequel le moyen de fixation (1210) comprend un prolongement (1220), dans lequel le prolongement (1220) présente une pluralité de saillies contre-dépouillées (1230) qui sont disposées de manière circonférentielle sur la surface d'enveloppe du prolongement (1220) de sorte que les saillies contre-dépouillées (1230) s'engrènent dans la région d'accouplement (1250) d'un pendant de prolongement (1240) lorsque le prolongement (1220) est relié au pendant de prolongement (1240), dans lequel le pendant de prolongement (1240) est réalisé en tant que manchon de médicament.

13. Procédé de fabrication d'un moyen de fixation selon les revendications 8 à 12 avec les étapes :
mise à disposition d'un substrat (10) en nitinol ;
traitement électrochimique d'une surface du substrat (10) pour la génération d'une pluralité de saillies contre-dépouillées (20) à la surface du substrat (10) ; et
connexion du moyen de fixation à un support de médicament en tant que pendant.

14. Procédé selon la revendication 13, dans lequel le traitement électrochimique s'effectue à l'aide d'un champ d'électrodes (30), dans lequel des électrodes (32) du champ d'électrodes (30) présentent des flancs latéraux isolés.
